# EUROPEAN PATENT APPLICATION

(11) **EP 1 829 977 A1**
(43) Date of publication of application: **05.09.2007**
(21) Application number: 05806833.9
(22) Date of filing: 18.11.2005
(51) Int. Cl.: C12Q 1/48, C12Q 1/68, C12N 15/09

(54) **METHOD OF DIAGNOSING THE RISK OF THERMOLABILE PHENOTYPE DISEASES BY USING GENE**

(30) Priority: 19.11.2004 JP 2004335958; 19.11.2004 JP 2004335966
(71) Applicant: OTSUKA PHARMACEUTICAL CO., LTD., Chiyoda-ku Tokyo 101-8535 (JP); The University of Tokushima, Tokushima-shi, Tokushima 7708501 (JP)
(72) Inventor: KIDO, Hiroshi, The University of Tokushima, Tokushima-shi, Tokushima 770-8503 (JP); KINOSHITA, Moritoshi, Otsuka Pharmaceut. Co., Ltd., Osaka-shi, Osaka 540-0021 (JP); MIZUGUCHI, Hiroshi, Otsuka Pharmaceutical Co., Ltd, Tokushima-shi, Tokushima 771-0192 (JP); TAKAHASHI, Norihiko, Otsuka Pharmaceut. Co., Ltd., Tokushima-shi, Tokushima 771-0192 (JP)
(74) Representative: HOFFMANN EITLE
(86) International application number: PCT/JP2005/021294
(87) International publication number: WO 2006/054722

(57) **Abstract**

The invention relates to a method of diagnosing a risk of a thermolabile phenotype disease including or caused by influenza encephalitis/encephalopathy, Reye's syndrome, RS virus infectious disease, adenovirus infectious disease, rhinovirus infectious diseases, bastard measles, Japanese encephalitis, malaria infectious disease, Kawasaki disease and sudden infant death syndrome, **characterized by** examining whether or not an enzymatic activity of at least one enzyme involved in any of various transporters, carnitine cycle, long-chain β oxidation cycle, medium-chain/short-chain β oxidation cycle, electron transfer, synthesis of a ketone and production of ATP involved in energy metabolism in mitochondria is significantly lower compared with healthy subjects at 39°C or higher when referring the enzymatic activity at 37°C as to 100%.

## Description

### Technical Field

The present invention relates to a method of diagnosing the risk of thermolabile phenotype diseases. The thermolabile phenotype disease means a constitution where a possibility that an aftereffect is left based on lethal multiple organ failure or central nervous system disorder capable of being induced by high fever is significantly higher compared with healthy subjects, and the risk of such a thermolabile phenotype disease can be diagnosed in the present invention.

### Background Art

In children, febrile convulsion often occurs upon running a fever at 39°C or higher, among them, there is a syndrome of undetermined cause, where serious central nervous symptom occurs by being triggered with persistent high fever, resulting in death with failure of liver, heart, kidney and the like, and methods of preventing, diagnosing and treating it remained unclear.

Non-patent literature 1 is a literature in which mutations of CPTII and biochemical functions thereof were studied, and a oxidation rate of palmitate at 37°C and 41°C was examined. However, there is no description for the association of functional changes of CPTII by temperature change with specific diseases, and in particular, no association of CPTII with influenza encephalitis/encephalopathy and Reye's syndrome having the central nervous symptoms is suggested.

Non-patent literature 2 has suggested involvement of activity decrease of CPTI and CPTII in Reye's syndrome, but the relation between the temperature and the activity of these enzymes is not described.

Non-patent literature 3 has described the association of severity of influenza encephalitis/encephalopathy with polymorphism of CYP2C9, but the association of the temperature with the enzyme activity is not disclosed.
Non-Patent literature 1: S.E. Olpin, et al. J. Inherit. Metab. Dis., 26: 543-557, 2003
Non-Patent literature 2: H. Abe, Chiba Igaku 74:9-16, 1998
Non-Patent literature 3: T. Funato et al., Rinsho Byori, 50(2):140-145, 2002

### DISCLOSURE OF THE INVENTION

### PROBLEM TO BE SOLVED BY THE INVENTION

It is an object of the present invention is to in advance diagnose (forecast) the risk of thermolabile phenotype diseases where a severe central nervous symptom occurs by being triggered with onset of fever.

### MEANS FOR SOLVING THE PROBLEM

It has been revealed by study of the present inventors that a genetic variation leading to rapid loss due to high fever of an enzymatic activity of a fatty acid metabolic enzyme in mitochondria essential for energy production *in vivo* is potentially present at high frequency in thermolabile phenotype diseases such as influenza encephalitis/encephalopathy with severe central nervous symptom and cerebral edema caused by being triggered with influenza infection. In this genetic variation, at normal body temperature, there is no abnormality in the enzymatic activity or even when the decrease of the enzymatic activity is observed, it is slight, there is typically no symptom and unless getting a high fever, no symptom often appears. Further, in many patients, the genetic variation is heterozygous, which means that a half of the enzyme is normal and the other half of the enzyme is a mutant, which are mixed in the patient. Thus, the patient often passes over in daily life without observed abnormality. Separately from this, there is a congenital metabolic disorder where the patient has congenitally the genetic variation in these fatty acid metabolic enzymes and the enzyme activity is remarkably decreased. In this case, it has been reported that within several months after birth, the patient exhibits Reye's syndrome without influenza infection leading to death with cerebral edema and multiple organ failure (Tamaoki Y., Kimura M., Hasegawa Y., Iga M., Inoue M., Yamagishi S., Brain Dev., 2002;24;675-80). In the thermolabile phenotype diseases including influenza encephalitis/encephalopathy, it has been revealed that the disease is caused by not such a severe enzyme deficiency but the genetic variation unstabilized by fever, which underlies in the disease.

From this, it is speculated that the possibility that the disease is caused by the same reason is high in a group of diseases in addition to influenza encephalitis/encephalopathy, where the multiple organ failure is induced by impairing the systematic energy production system with high fever, e.g., sudden infant death syndrome described to be induced by unknown cause, various infectious diseases other than influenza encephalitis/encephalopathy, e.g., patients exhibiting the severity and the multiple organ failure due to RS virus infectious diseases, adenovirus infectious diseases, rhinovirus infectious diseases, bastard measles, Japanese encephalitis and malaria infectious diseases, Reye's syndrome which occurs when dosing aspirin, voltaren or mefenamic acid, and Kawasaki disease. Thus, the present inventors have made these diseases the "thermolabile phenotype diseases", developed a new method of precisely diagnosing the mutation of thermolabile genes which cause the onset, and completed the present invention.

The present invention provides the following method of diagnosing the risk and a diagnostic kit.
[1] A method of diagnosing a risk of a thermolabile phenotype disease including or caused by influenza encephalitis/encephalopathy, Reye's syndrome, RS virus infectious disease, adenovirus infectious disease, rhinovirus infectious diseases, bastard measles, Japanese encephalitis, malaria infectious disease, Kawasaki disease and sudden infant death syndrome, characterized by examining whether or not an enzymatic activity of at least one enzyme involved in any of various transporters, carnitine cycle, long-chain β oxidation cycle, medium-chain/short-chain β oxidation cycle, electron transfer, synthesis of a ketone and production of ATP involved in energy metabolism in mitochondria is significantly lower compared with healthy subjects at 39°C or higher when referring the enzymatic activity at 37°C as to 100%.
[2] The method according to [1] wherein the thermolabile phenotype disease is influenza encephalitis/encephalopathy.
[3] The method according to [1] characterized in that a degree of the decreased enzymatic activity at 39°C compared with the enzymatic activity at 37°C is forecasted by examining polymorphism selected from the group consisting of SNP, insertion and deletion in a gene encoding the enzyme.
[4] The method according to [1] wherein the enzyme involved in any of various transporters, carnitine cycle, long-chain β oxidation cycle, medium-chain/short-chain β oxidation cycle, electron transfer, synthesis of a ketone and Production of ATP is at least one enzyme shown in the following Table.

**Table 1**

| | Names of enzymes |
|---|---|
| Transporter | Tricarboxylate transport protein (TCT) |
| | Voltage-dependent anion channel (VDAC) |
| | Adenine nucleotide Transporter (ANT) |
| | Carnitine Transporter (CRNT; OCTN2; SLC22A5) |
| | Fatty acid plasma membrane transporter (LCFAT) |
| Carnitine cycle | Acyl-CoA synthetase (ACS) |
| | Carnitine palmitoyl transferase 1 liver form (CPT1A; CPT1) |
| | Carnitine palmitoyl transferase 1 muscle form (CPT1b; CPT1B) |
| | Carnitine palmitoyl transferase 2 (CPT2) |
| | Carnitine/Acylcarnitine translocase (CACT1; SLC25A20) |
| | Very-long-chain acyl-CoA dehydrogenase (VLCAD) |
| Long-chain β oxidation cycle | TFP mitochondrial trifunctional protein alpha-subunit (TFPα;HADHA) |
| | TFP mitochondrial trifunctional protein beta-subunit (TFPβ; HADHB) |
| | Long-chain acyl-CoA dehydrogenase (LCAD) |
| Medium-chain/short-chain oxidation cycle | Medium-chain acyl-CoA dehydrogenase (MCAD) |
| | Medium-chain acyl-CoA thiolase (MCKAT) |
| | Short-chain acyl-CoA dehydrogenase (SCAD) |
| | Short-chain enoyl-CoA hydratase (SCEH) |
| | Short-chain hydroxyacyl-CoA dehydrogenase (SCHAD) |
| | Short-chain acyl-CoA thiolase (SCKAT) |
| Electron transfer | Electron Transfer Flavoprotein Alpha subunit ;ETFA |
| | Electron Transfer Flavoprotein beta subunit ;ETFB |
| | Electron Transfer Flavoprotein dehydrogenase (ETFDH) |
| | NADH-ubiquinone reductase complex(Complex I) |
| | Succinate-ubiquinone reductase (Complex II) |
| | Ubiquinol-cytochrome-c reductase (Complex III) |
| | Cytochrome-c oxidase (Complex IV) |
| Production of ATP | ATP synthase |
| | Uncoupling protein (UCP) |
| Synthesis of a ketone | Hydroxymethylglutaryl-CoA synthetase 1, 2 (HMGCS1 or 2; HMGS1 or 2) |
| | Hydroxymethylglutaryl-CoA ligase (HMGCL; HMGL) |

[5] The method according to [4] wherein the enzyme is at least one selected from the group consisting of CPTII, ETFA, ETFB, ETFDH, HADHB, HMGCS, VLCAD, LCAD and HADHA.
[6] The method according to [1] characterized in that concerning CPTII, ETFA, ETFB, HADHA, HADHB, HMGCS, VLCAD, LCAD and ETFDH, SNP of genes at one or two positions in the following 22 positions or genes being in linkage disequilibrium therewith is examined and a combination of two or more genetic polymorphisms or haplotypes is utilized.

**Table 2**

| Enzyme name | Position of SNP | |
|---|---|---|
| CPT2 | (EXON4) | 1055 |
| CPT2 | (EXON4) | 1102 |
| ETFA | (INTRON10) | +642 |
| ETFB | (EXON1) | -320 |
| ETFB | (EXON3) | -113 |
| ETFB | (EXON8) | 447 |
| ETFB | (EXON8) | 461 |
| HADHA | (EXON6) | 474 |
| HADHA | (INTRON6) | +26 |
| HADHA | (INTRON6) | +32 |
| HADHA | (EXON18) | 2519 |
| HADHA | (EXON18) | 2619 |
| HADHB | (EXON2) | 4 |
| HADHB | (INTRON12) | -14 |
| HADHB | (INTRON14) | +4 |
| HADHB | (INTRON14) | -26 |
| HADHB | (EXON17) | 1607 |
| HMGCS | (INTRON8) | -37 |
| HMGCS | (INTRON8) | +53 |
| VLCAD | (INTRON8) | +33 |
| LCAD | (EXON9) | 997 |
| ETFDH | (EXON13) | 1989 |

[7] The method according to [1] wherein the risk of the thermolabile phenotype disease is diagnosed based on whether having the combination of any SNP in the following (1) to (8) or not.

**Table 3**

| Combination | Enzyme | Polymorphism position (base number) | Genotype | Risk |
|---|---|---|---|---|
| (1) | ETFB | 447 | C/T | Large |
| | CPT2 | 1102 | G/A | |
| (2) | ETFA | +642 | C/C | Large |
| | ETFB | -113 | G/T | |
| (3) | ETFA | +642 | C/C | Large |
| | CPT2 | 1055 | G/G or G/T | |
| (4) | HADHB | 4 | -/ACT or ACT/ACT | Large |
| | HMGCS | -37 | C/T or T/T | |
| (5) | VLCAD | +33 | T/G or G/G | Large |
| | LCAD | 997 | A/A | |
| (6) | CPT2 | 1055 | T/G or G/G | Large |
| | CPT2 | 1102 | G/A or A/A | |
| (7) | HADHB | 4 | -/ACT or ACT/ACT | Large |
| | ETFDH | 1989 | G/T | |
| (8) | LCAD | 997 | A/C or C/C | Small |
| | HADHA | 2619 | G/G | |
| | HMGCS | +53 | T/T | |

[8] The method according to any of [1] to [7] wherein single nucleotide polymorphism (SNP) is detected by at least one method selected from the group consisting of a nucleotide direct base sequencing method, an allele specific oligonucleotide (ASO)-dot blotting analysis, a single base primer extension method, a PCR-single strand conformation polymorphism (SSCP) analysis, a PCR-restriction enzyme fragment length polymorphism (RFLP) analysis, an invader method, a quantitative real-time detection method and a single nucleotide polymorphism detection method (mass array) using a mass spectrometer.
[9] The method according to [3] characterized in that the polymorphism selected from the group consisting of SNP, insertion and deletion in the gene encoding the enzyme is measured using a solid phase support to which at least one corresponding probe has been immobilized.
[10] A diagnostic kit for diagnosing a risk of a thermolabile phenotype disease comprising primers, probes, a dNTP mix, reverse transcriptase, DNA polymerase and buffer capable of detecting one or a combination of two or more specific polymorphisms of an enzyme involved in β oxidation fatty acid metabolic system in mitochondria.

### EFFECTS OF THE INVENTION

According to the present invention, it becomes possible to diagnose the risk of the thermolabile phenotype disease and manage so that the thermolabile phenotype disease is not developed by preventing the disease with high fever or giving an early treatment in the case of having the disease when those diagnosed to have the high risk are children particularly including infants.

### BRIEF DESCRIPTION OF THE DRAWINGS

FIG. 1 is a graph showing distribution of acylcarnitine [(C_{16:0}+C_{18:1})/C₂] ratio in IAE patients having a high fever/convulsion or normal body temperature. The acylcarnitine ratios in the IAE patients, familial relatives of the patient No. 21 and one volunteer family not infected with influenza were analyzed. A maximum cutoff range of 0.048 and a high risk patient range of 0.09 or more are represented by a thin dotted line and a bold dotted line, respectively. +: Lethal, closed triangle: aftereffects, and x: elder sister of patient No. 21;
FIG. 2 is a graph showing change with time of CPTII activity in liver homogenates from the control and the patient No. 21 at 37°C and 41°C. Data are represented by mean ± SD (n=6);
FIG. 3 is a graph showing the comparison of activities of WT CPTII and mutant CPTII excessively expressed in COS-7 cells at 37°C and 41°C. The CPTII activity of WT (FVM-CPTII), F352C (CVM-CPTII), V368I (FIM-CPTII) and F352C + V368I (CIM-CPTII) was measured at 37°C and 41°C. Data are represented by mean ± SD (n=5);
FIG. 4 shows genes encoding enzymes usable for risk diagnosis and their SNP or insertions/deletions and their regions and positions, genotypes and phenotypes, and sequences proximal thereto; and
FIG. 5 shows polymorphisms observed at significantly high frequency in the patients.

### BEST MODES FOR CARRYING OUT THE INVENTION

Racial differences have been reported for gene mutation in an enzyme group for energy metabolic disorder. It has been known that in Japan the gene mutation frequently occurs in the enzyme group involved in long-chain fatty acid metabolism of β oxidation cycle and the enzyme group involved in electron transfer whereas in Caucasian in Europe and US, the gene mutation frequently occurs in the enzyme group involved in medium-chain fatty acid metabolism. It is thought that the thermolabile gene mutation induced by high fever also has a background of similar incidence frequencies of the gene mutation.

As the enzyme groups capable of being subjected to the thermolabile phenotype disease, the followings are exemplified.
1) Transporters: carnitine transporters (OCTN2, OCTN), fatty acid transporter (CD36), voltage dependent anion channel (VDAC), carnitine/acylcarnitine transferase (CACT1), tricarboxylic acid transport protein (TCT) and adenine nucleotide transporter (ANT).
2) Carnitine cycle and related enzymes: acyl-CoA synthase (ACS), carnitine palmitoyl transferase 1 liver type (CPT1A, CPT1), carnitine palmitoyl transferase muscle type (CPT1b, CPT1B), carnitine palmitoyl transferase 2 (CPT2), carnitine/acylcarnitine translocase (CACT1, SLC25A20).
3) Long-chain β oxidation cycle: very long-chain acyl-CoA dehydrogenase (VLCAD), TFP mitochondrial trifunctional protein α-subunit (TFPα, HADHA), TFP mitochondrial trifunctional protein β-subunit (TFPβ, HADHA),
4) Medium-chain/short-chain β oxidation cycle: long-chain acyl-CoA dehydrogenase (LCAD), medium-chain acyl-CoA dehydrogenase (MCAD), medium-chain acyl-CoA thiolase (LCKAT), short-chain acyl-CoA dehydrogenase (SCAD), short-chain enoyl CoA hydratase (SCEH), short-chain deoxy-acyl CoA dehydrogenase (SCHAD), short-chain acyl CoA thiolase (SCKAT).
5) Electron transfer: electron transfer flavoprotein α-subunit (ETFA), electron transfer flavoprotein β-subunit (ETFB), electron transfer flavoprotein dehydrogenase (ETFDH), NADH-ubiquinone reductase complex (complex I), succinate-ubiquinone reductase (complex II), ubiquinone cytochrome-c reductase (complex III), cytochrome-c oxidase (complex IV).
6) Production of ATP: ATP synthase, uncoupling protein (UCP).
7) Synthesis of a ketone: hydroxymethylglutaryl-CoA-synthase or 2 (HMGCS1 or 2; HMGS1 or hydroxymethylglutaryl-CoA-ligase (HMGCL, HMGL).

Herein, the "method of diagnosing the risk" means forecasting the risk that the lethal or aftereffect-leaving multiple organ failure or central nervous symptom is developed when the patient has had the thermolabile phenotype disease including or caused by influenza encephalitis/encephalopathy, Reye's syndrome, RS virus infectious disease, adenovirus infectious disease, rhinovirus infectious diseases, bastard measles, Japanese encephalitis, malaria infectious disease, Kawasaki disease and sudden infant death syndrome and has gotten a high fever (typically 39°C or higher, further 40°C or higher and particularly 40°C or higher).

Therefore, it is important to perform this risk diagnosis or forecast before having the thermolabile phenotype disease. Adults including the elderly also have the thermolabile phenotype disease, but in particular when infants and children have the thermolabile phenotype disease, they easily develop the lethal or aftereffect-leaving multiple organ failure or central nervous symptom. Therefore, the subjects subjected to the risk diagnosis of the present invention are not particularly limited as long as they are human beings, but they are preferably children, for example aged 18 years or younger, further 15 years or younger and particularly 12 years or younger, and the infants (particularly babies) are preferable in terms of inhibiting the development of the thermolabile phenotype disease.

When diagnosed to have the high possibility to have the thermolabile phenotype disease as a result of risk diagnosis in human beings including the children (particularly the infants), it is important to do not to get the disease with high fever associated with the thermolabile phenotype disease. In the case of viral or bacterial infectious diseases, gargle, washing hands, use of face guards and administration of preventive drugs such as vaccine are included. When getting the disease with high fever capable of causing the thermolabile phenotype disease, it is important to control the body temperature to lower than 41°C, further lower than 40°C, particularly lower than 39°C and more preferably lower than 38°C by rapidly administering a therapeutic drug or a safe antipyretic agent.

The thermolabile phenotype disease herein includes influenza encephalitis/encephalopathy, Reye's syndrome, RS virus infectious disease, adenovirus infectious disease, rhinovirus infectious diseases, bastard measles, Japanese encephalitis, malaria infectious disease, Kawasaki disease and sudden infant death syndrome.

The present invention is based on finding that the lethal or aftereffect-leaving severe multiple organ failure or central nervous symptom is caused by rapidly decreasing the enzyme activity in the β oxidation fatty acid metabolic system in the mitochondria in a feverish condition.

When such a multiple organ failure or central nervous symptom occurs, the enzyme activity in the healthy subject is decreased by about 5 to 10% at 41°C when the enzyme activity at normal body temperature (e.g., 37°C) is referred to as 100%. When this enzyme activity is decreased by more than 50%, there is the possibility that the multiple organ failure/central nervous symptom appears. As a decrease rate is increased to 60% or more, 70% or more, 80% or more and 90% or more, the probability of the development of the multiple organ failure/central nervous symptom is increased. When the decrease rate of the enzyme activity at 39°C or higher exceeds 80%, the possibility that the multiple organ failure/central nervous symptom is developed is extremely high.

The decrease of the enzyme activity can be evaluated by examining a nucleotide sequence of an enzyme of the subject subjected to the diagnosis of the present invention, producing the enzyme based on the nucleotide sequence by gene engineering techniques and comparing the enzyme activities after treating the enzyme at 37°C and 39°C or higher (e.g., 39°C, 40°C, 41°C) for a certain time period. A risk size for the thermolabile phenotype disease can be evaluated based on this sequence by examining the nucleotide sequence of the enzyme whose activity was significantly decreased compared with that from the healthy subject at 39°C or higher (e.g., 39°C, 40°C, 41°C). For example, in the case of the enzyme being CPTII, when having both polymorphisms of F352C and V368I, the possibility that the severe central nervous symptom is caused upon running a fever is high, and it is possible to diagnose as the thermolabile phenotype disease.

The degree of the decreased enzyme activity at 39°C or higher compared with the enzyme activity at 37°C can be predicted by examining SNP in the gene encoding the enzyme.

The enzymes particularly useful for the risk diagnosis include CPTII, ETFA, ETFB, ETFDH, HADHB, HMGCS, VLCAD, LCAD and HADHA.

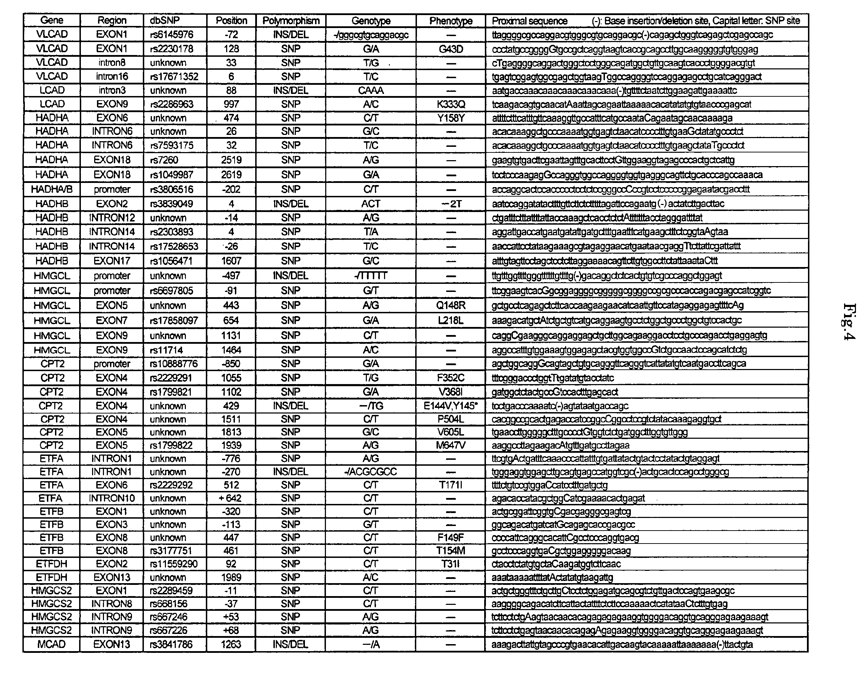

The genes encoding the enzymes usable for the risk diagnosis, their SNPs or insertions/deletions and their regions and positions, genotypes and phenotypes are summarized in Table 4, and they are shown including the sequences proximal to SNPs in FIG. 4.

**Table 4**

| Gene | Region | dbSNP | Position | Polymorphism | Genotype | Phenotype |
|---|---|---|---|---|---|---|
| VLCAD | EXON1 | rs6145976 | -72 | INS/DEL | -/gggcgtgcaggacgc | - |
| VLCAD | EXON1 | rs2230178 | 128 | SNP | G/A | G43D |
| VLCAD | intron8 | unknown | +33 | SNP | T/G | - |
| VLCAD | intron16 | rs17671352 | +6 | SNP | T/C | - |
| LCAD | intron3 | unknown | +88 | INS/DEL | CAAA | - |
| LCAD | EXON9 | rs2286963 | 997 | SNP | A/C | K333Q |
| HADHA | EXON6 | unknown | 474 | SNP | C/T | Y158Y |
| HADHA | INTRON6 | unknown | +26 | SNP | G/C | - |
| HADHA | INTRON6 | rs7593175 | +32 | SNP | T/C | - |
| HADHA | EXON18 | rs7260 | 2519 | SNP | A/G | - |
| HADHA | EXON18 | rs1049987 | 2619 | SNP | G/A | - |
| HADHA/B | promoter | rs3806516 | -202 | SNP | C/T | - |
| HADHB | EXON2 | rs3839049 | 4 | INS/DEL | ACT | 1-2Tins |
| HADHB | INTRON12 | unknown | -14 | SNP | A/G | - |
| HADHB | INTRON14 | rs2303893 | +4 | SNP | T/A | - |
| HADHB | INTRON14 | rs17528653 | -26 | SNP | T/C | - |
| HADHB | EXON17 | rs1056471 | 1607 | SNP | G/C | - |
| HMGCL | promoter | unknown | -497 | INS/DEL | -/TTTTT | - |
| HMGCL | promoter | rs6697805 | -91 | SNP | G/T | - |
| HMGCL | EXON5 | unknown | 443 | SNP | A/G | Q148R |
| HMGCL | EXON7 | rs17858097 | 654 | SNP | G/A | L218L |
| HMGCL | EXON9 | unknown | 1131 | SNP | C/T | - |
| HMGCL | EXON9 | rs11714 | 1464 | SNP | A/C | - |
| CPT2 | promoter | rs10888776 | -850 | SNP | G/A | - |
| CPT2 | EXON4 | rs2229291 | 1055 | SNP | T/G | F352C |
| CPT2 | EXON4 | rs1799821 | 1102 | SNP | G/A | V3681 |
| CPT2 | EXON4 | unknown | 429 | INS/DEL | -/TG | E144V,Y145* |
| CPT2 | EXON4 | unknown | 1511 | SNP | C/T | P504L |
| CPT2 | EXON5 | unknown | 1813 | SNP | G/C | V605L |
| CPT2 | EXON5 | rs1799822 | 1939 | SNP | A/G | M647V |
| ETFA | INTRON1 | unknown | -776 | SNP | A/G | - |
| ETFA | INTRONI | unknown | -270 | INS/DEL | -/ACGCGCC | - |
| ETFA | EXON6 | rs2229292 | 512 | SNP | C/T | T171I |
| ETFA | INTRON10 | unknown | +642 | SNP | C/T | - |
| ETFB | EXON1 | unknown | -320 | SNP | C/T | - |
| ETFB | EXON3 | unknown | -113 | SNP | G/T | - |
| ETFB | EXON8 | unknown | 447 | SNP | C/T | F149F |
| ETFB | EXON8 | rs3177751 | 461 | SNP | C/T | T154M |
| ETFDH | EXON2 | rs11559290 | 92 | SNP | C/T | T31I |
| ETFDH | EXON13 | unknown | 1989 | SNP | A/C | - |
| HMGCS2 | EXON1 | rs2289459 | -11 | SNP | C/T | - |
| HMGCS2 | INTRON8 | rs668156 | -37 | SNP | C/T | - |
| HMGCS2 | INTRON9 | rs667246 | +53 | SNP | A/G | - |
| HMGCS2 | INTRON9 | rs667226 | +68 | SNP | A/G | - |
| MCAD | EXON13 | rs3841786 | 1263 | INS/DEL | -/A | - |

The risk diagnosis of the present invention can be carried out by single nucleotide polymorphism using the polymorphism of HADHA and HADHB at positions in the following Table 5.

**Table 5**

| Gene | Region | Position | Type of Polymorphism | Genotype | Phenotype |
|---|---|---|---|---|---|
| HADHA | EXON6 | 474 | SNP | C>T | Y158Y |
| HADHA | INTRON6 | +26 | SNP | G>C | - |
| HADHA | INTRON6 | +32 | SNP | T>C | - |
| HADHA | EXON18 | 2519 | SNP | A>G | - |
| HADHA | EXON18 | 2619 | SNP | G>A | - |
| HADHB | EXON2 | 4 | INS/DEL | ->ACT | 1-2Tins |
| HADHB | INTRON12 | -14 | SNP | A>G | - |
| HADHB | INTRON14 | +4 | SNP | T>A | - |
| HADHB | INTRON14 | -26 | SNP | T>C | - |
| HADHB | EXON17 | 1607 | SNP | G>C | - |

When diagnosed by combining two or more polymorphisms, the combinations shown in the following Table 6 are preferable.

**Table 6**

| Combination | Enzyme | Polymorphism position (base number) | Genotype | Risk |
|---|---|---|---|---|
| (1) | ETFB | 447 | C/T | Large |
| | CPT2 | 1102 | G/A | |
| (2) | ETFA | +642 | C/C | Large |
| | ETFB | -113 | G/T | |
| (3) | ETFA | +642 | C/C | Large |
| | CPT2 | 1055 | G/G or G/T | |
| (4) | HADHB | 4 | -/ACT or ACT/ACT | Large |
| | HMGCS | -37 | C/T or T/T | |
| (5) | VLCAD | +33 | T/G or G/G | Large |
| | LCAD | 997 | A/A | |
| (6) | CPT2 | 1055 | T/G or G/G | Large |
| | CPT2 | 1102 | G/A or A/A | |
| (7) | HADHB | 4 | -/ACT or ACT/ACT | Large |
| | ETFDH | 1989 | G/T | |
| (8) | LCAD | 997 | A/C or C/C | Small |
| | HADHA | 2619 | G/G | |
| | HMGCS | +53 | T/T | |

The risk can also be determined using another allele in linkage disequilibrium with the allele having each single nucleotide polymorphism.

Each single nucleotide polymorphism is often taken from a parent to a progeny together with the adjacent genetic polymorphism, and a relationship between those alleles is referred to as the linkage disequilibrium. The linkage disequilibrium tends to be weakened as the distance between the alleles gets longer. Therefore, in the proximal genes, the strong linkage disequilibrium is often observed. When the allele shown herein and the adjacent allele are in the relationship of the strong linkage disequilibrium, the frequency and the appearance pattern of the genetic polymorphism become sometime the same, and it is possible to predict the genetic polymorphism of the present Example. Meanwhile, without limiting to the gene described herein, by analyzing the other adjacent gene, it becomes possible to utilize this for the risk diagnosis of the present invention. Even if there is the strong linkage disequilibrium, the risk diagnosis can be sometimes performed.

For example, in many cases, HADHA and HADHB are in linkage disequilibrium. Therefore, it is possible to perform the risk diagnosis by utilizing the genetic polymorphism adjacent to this gene.

Genetic polymorphisms in linkage disequilibrium are restricted, and limited arrangement of the genetic polymorphisms is formed. The combination of the arrangements of these genetic polymorphisms on one chromosome is referred to as a haplotype.

The risk diagnosis of the present invention can be performed using a diagnostic kit comprising primers, probes, a dNTP mix, reverse transcriptase, DNA polymerase and buffer capable of detecting one or a combination of two or more specific polymorphisms of the enzyme involved in β oxidation fatty acid metabolic system in mitochondria.

The present invention also relates to such a diagnostic kit.

The method of diagnosing the risk of the present invention can also be performed using other methods.

The detection of the genetic polymorphism is not limited to the present method, and can be performed using a direct sequencing method, a single base primer extension method, a PCR-single strand conformation polymorphism (SSCP) analysis, a PCR-restriction enzyme fragment length polymorphism (RFLP) analysis, an invader method and a mass spectrometer. In the method of diagnosing the risk, not limiting to DNA materials, the genetic polymorphism can be analyzed using RNA materials, and the gene expression can be quantified by a quantitative real-time PCR detection method. The genetic polymorphism resulting in amino acid substitution can also be analyzed by an antibody radioimmunoassay and an enzyme linked immunosorbent assay (ELISA). In the above methods, to efficiently perform them, the polymorphism is detected by immobilizing to a solid phase support such as beads, chips and membranes.

### EXAMPLES

The present invention will be described in more detail below using Examples.

### Example 1

Specific experimental methods performed by the present inventor and their results are shown below.

### Materials

31 Specimens from patients with influenza-associated encephalopathy, one specimen from the patient suspected to have influenza-associated encephalopathy (approved by Ethics Committee in each facility, and an informed consent was obtained from all patients), and 100 specimens from healthy subjects.

### Methods

Each gene was amplified using genomic DNA extracted from 31 specimens from patients with influenza-associated encephalopathy, one specimen from the patient suspected to have influenza-associated encephalopathy and 100 specimens from healthy subjects, and using primers for PCR (PCR-F, PCR-R) shown in Table A. A base sequence was analyzed using each amplified gene and the primers (Seq-F, Seq-R) shown in Table A by a direct sequencing method. Database of NCBI (http://www.nlm.nih.ncbi.gov, http://www.ncbi.nlm.nih.gov/projects/SNP/) and HGMD (http://www.hgmd.cf.ac.uk/hgmd0.html) were used as references for genetic polymorphisms. In Table 4, the numbers beginning with rs are reference numbers in the database, and the genetic polymorphism described to be unknown is the genetic polymorphism not registered to NCBI. Concerning CPTII, ETFA, ETFB, ETFDH, VLCAD, LCAD, HADHA and HADHB, the gene analysis was possible in 31 specimens from the patients. The other genes could be analyzed in 27 specimens. Meanwhile, for the healthy subject specimens, CPTII, ETFA, ETFB and ETFDH could be analyzed in 100 specimens, but the genes other than them could be analyzed in 99 specimens from the healthy subjects. Those having the allele frequently observed in the healthy subjects in homozygote, those having another allele in homozygote and those having these alleles in heterozygote were defined as W, M and H, respectively (W, H and M in Table 4 and FIG. 4).

**Table A**

| | | | | |
|---|---|---|---|---|
| *CPT2* | | | | |
| Name | Sequence (5' To 3') | | | |
| CPT2-F | AGACTTCCTAGAGCCGGAG | | PCR-F | Seq-F |
| CPT2-R | AAACGTGATTGGAATCTGATAAC | | PCR-R | Seq-R |
| CTP2exllAF | CCTACTAGTGGGCGGGGCCTGTCAGTGAGC | | PCR-F | Seq-F |
| CTP2ex11AR | GGAAACGGGTTCACTAGAGAGTCATGAGTGACTG | | PCR-R | Seq-R |
| CPT2e1s1 | AGCCAGTCCGGGGCG | | PCR-F | Seq-F |
| CPT2e1R | CGATCCTGGGACGGCGG | | PCR-R | Seq-R |
| CPT2e2F | ATTAACCTCTTCCATATACTGTCAGCC | | PCR-F | Seq-F |
| CPT2e2R | CCACCACTACTTGCCAGCCT | | PCR-R | Seq-R |
| CPT2e3F | CATGAACCTAAAAATCATGTATTCCCTA | | PCR-F | Seq-F |
| CPT2e3R | CATTATGGAGGGCTCTGGGAG | | PCR-R | Seq-R |
| CPT2e4F | GGGACAGCATTAACATTTTATGTTATTT | | PCR-F | Seq-F |
| CPT2e4R | CCAAGCACTGAGGACAAGACC | | PCR-R | Seq-R |
| CPT2e4s1 | CTTCACTGATGACAAGGCCAGAC | | | Seq-F |
| CPT2e4s2 | TGTCATCAGTGAAGAGTTCATCCC | | | Seq-R |
| CPT2e4s3 | CTCTACTGCCGTCCACTTTGAGC | | | Seq-F |
| CPT2e4s4 | CATTAAAAAATCTGAGCACTGCCAC | | | Seq-R |
| CPT2e4s5 | GCCACCTACGAGTCCTGTAGCA | | | Seq-F |
| CPT2e4s6 | GCGGATGGTCTCAGTGCG | | | Seq-R |
| CPT2e5F | CCTTTTCCATCCTGAGACGCT | | PCR-F | Seq-F |
| CPT2e5R | AGATCTTTGTGAGGATTAGGAATTAGGT | | PCR-R | Seq-R |
| CPT2e5s1 | AGGCAAATCCATCAAAAGTTAACTTC | | | Seq-F |
| CPT2e5s2 | TTTCATGATGAGGAAGTGATGGTAG | | | Seq-R |
| CPT2e5s3 | GGCGACAGAGCGAGACTGTC | | | Seq-F |
| CPT2e5s4 | CACCCACTGGCTACACAGGC | | | Seq-R |
| Subtotal | | 24 | | |
| | | | | |
| *ETFA* | | | | |
| NAme | Sequence (5' To 3') | | | |
| ETFAe1F | CTCGCGAGCATTAGGTGACTG | | PCR-F | Seq-F |
| ETFAe1R | GCCGTCCCTGGGTTCG | | PCR-R | Seq-R |
| ETFAe2F | AAAAAAAACCCTGTGACAACATTCT | | PCR-F | Seq-F |
| ETFAe2R | TGCCATCTTTTCCTGTCTCTTGA | | PCR-R | Seq-R |
| ETFAe3F | GATTTTATGATCTGTTGATATTTATAGTGGTG | | PCR-F | Seq-F |
| ETFAe3R | TCAAGCGTAATTTAGACACTACATTTTTT | | PCR-R | Seq-R |
| ETFAe4F | TTTCCAGTAGAAAAAAATGTAGTGTCTAAATT | | PCR-F | Seq-F |
| ETFAe4R | TCTAACCCTTTCAAGCAGTGATATCA | | PCR-R | Seq-R |
| ETFAe5F | GTCTTCCATCACTATGTATGTGTGTTAACT | | PCR-F | Seq-F |
| ETFAe5R | TGGTTTGATTTAAAATTCTATCTTCAAGATTA | | PCR-R | Seq-R |
| ETFAe6F | CCAAGCATCAAAGGAGTGCTAGT | | PCR-F | Seq-F |
| ETFAe6R | GTCTATGAATTAAAAAAGTAAGAAACAAAACAAA | | PCR-R | Seq-R |
| ETFAe7F | TATAGAACTTGAAATCATAGTCATTGTTTTATTATG | | PCR-F | Seq-F |
| ETFAe7-261R | TAAAAAAAAAAATCAAAGTATTTTCAATTTA | | PCR-R | Seq-R |
| ETFAe8F | CAGAATGTATTTGATGTTTGCCTAATTT | | PCR-F | Seq-F |
| ETFAe8R | TTTCCAACAAAAAGGGAATATCTTTC | | PCR-R | Seq-R |
| ETFAe9F | CTAGCTGAATAATAGAGCTTGAAAAAAATG | | PCR-F | Seq-F |
| ETFAe9R | CAACAAATACATACAGTACTTATCCCCATA | | PCR-R | Seq-R |
| ETFAe10F | CATCCCAGAGCAGCAGTTCA | | PCR-F | Seq-F |
| ETFAe10R | AGTGGGCTCCTTGGCTCTC | | PCR-R | Seq-R |
| ETFAe11F | AAGACTTAAATTGCTAACAGCAAACACTT | | PCR-F | Seq-F |
| ETFAe11R | CCAAATCCTAAAATGTGGAAACGT | | PCR-R | Seq-R |
| ETFAe12F | GTCTGTGTGAAATGTTTTCTTGGTAAA | | PCR-F | Seq-F |
| ETFAe12R | ACCATTTCTGAGAGTTTCTCGGA | | PCR-R | Seq-R |
| ETFAe13F | CAGTGAAGGCTCTAGTTGCTTAATTACT | | PCR-F | Seq-F |
| ETFAe13R | CATGAACAAGAAAGGCAAAAAAATAA | | PCR-R | Seq-R |
| ETFA e10s1 | ACATTGCCTGCAGCATGGAG | | | Seq-F |
| ETFA e10s2 | TCGGTGGATGAAGTTCCGAG | | | Seq-R |
| ETFA e10s3 | TCATTGGCGGGAATCACCTG | | | Seq-F |
| ETFA e10s4 | GTGGGAGTGTAAGAACGTCCTCC | | | Seq-R |
| Subtotal | | 30 | | |
| | | | | |
| *ETFB* | | | | |
| Name | Sequence (5' To 3') | | | |
| ETFB-F | GGCTTCTGCTTTCTGCTG | | PCR-F | Seq-F |
| ETFB-R | ACTCAGGGATGTGGGAGAG | | PCR-R | Seq-R |
| ETFB-s1 | AGAGTGGAGAGACCGACG | | | Seq-F |
| ETFB-s2 | GGACCTGGAGAGAGAAGG | | | Seq-R |
| ETFBe1-F | GGCCGAACCCGTAGTGACT | | PCR-F | Seq-F |
| ETFBe1-R | CAGGCCCCTCCATCACC | | PCR-R | Seq-R |
| ETFBe2-21F | GGGCGGGTCTGAAGGG | | PCR-F | Seq-F |
| ETFBe2-R | GGCAGTCTCCTCCACCCTC | | PCR-R | Seq-R |
| ETFBe3-F | CCACCTTCCTCTCCCTGCTC | | PCR-F | Seq-F |
| ETFBe3-R | CGCTAAAAAAAGCCGTTCCTT | | PCR-R | Seq-R |
| ETFBe4-F | TGCGGGCTGACCCTGT | | PCR-F | Seq-F |
| ETFBe4-R | CGGGACTCAGGGATGTGG | | PCR-R | Seq-R |
| ETFBe5-2861F | CTGTGTGACCCCGGACAAG | | PCR-F | Seq-F |
| ETFBe5-3263R | GGGTCCAGACTTCCAGCCTC | | PCR-R | Seq-R |
| ETFBe-F | GTCCCCCTGAGGATAGCAGC | | PCR-F | Seq-F |
| ETFBe6-R | CCCGTATCTCCACCACCCT | | PCR-R | Seq-R |
| ETFBex7F | TCCATGAGCTCCACTGATTGTC | | PCR-F | Seq-F |
| ETFBex7R | AGTTTGAGGGATGAGGGCCTG | | PCR-R | Seq-R |
| ETFBe8-F | CTGAATTAGCCTCACCCACTCTC | | PCR-F | Seq-F |
| ETFBe8-306R | GTGCTTGCCACCCCCA | | PCR-R | Seq-R |
| ETFBe9-F | GCAGGAGTTCCACAGCCCT | | PCR-F | Seq-F |
| ETFBe9-R | TGAACCAAATAATGGGTCTCTAGGA | | PCR-R | Seq-R |
| Subtotal | | 22 | | |
| *ETFDH* | | | | |
| Name | Sequence (5' To 3') | | | |
| ETFDH-F | CACCAACTGTCTTTTAACCACTC | | PCR-F | Seq-F |
| ETFDH-R | GCGAGAGGTGCCTACAGC | | PCR-R | Seq-R |
| ETFDH-s1 | CGTCGTGCTGTGGGATTC | | | Seq-F |
| ETFDH-s2 | CACCTCCTTCTCGCTCTG | | | Seq-R |
| ETFDHe1-F | CGCAGAGCGAGAAGGAGGT | | PCR-F | Seq-F |
| ETFDHe1-R | CCTAACTCCTCCACTTATCCCCA | | PCR-R | Seq-R |
| ETFDHe2-F | CAAAGTGTTCACCTAGGAAAGATTATAGTATATT | | PCR-F | Seq-F |
| ETFDHe2-R | AAGTATCCAGAAAAGTCTCAGGAATTG | | PCR-R | Seq-R |
| ETFDHe3-F | TCATGTGATTATTGGGTTATATTAATCCC | | PCR-F | Seq-F |
| ETFDHe3-R | CCAGTAATTTGGGAACAATTACTGAA | | PCR-R | Seq-R |
| ETFDHe4-F | ACACTTGCAAATATAAACTAAAAACATTTCTT | | PCR-F | Seq-F |
| ETFDHe4-R | AATGTAGTTCCTTCCAGCTGTGG | | PCR-R | Seq-R |
| ETFDHe5-F | GTGACCATCAATGTAGCACTTATGTAGA | | PCR-F | Seq-F |
| ETFDHe5-R | TCTCCACTTATTAAAAAAAGAGAGTTCCTATA | | PCR-R | Seq-R |
| ETFDHe6-F | GTTTTTTTCAAGATTATTATGAATTCTAAGGTATT | | PCR-F | Seq-F |
| ETFDHe6-R | TCTTTCTGTTGTTTGTCGCTTTATTAAG | | PCR-R | Seq-R |
| ETFDHe7-F | GAATGTGAATGTATTTTAAATTGTCAAATG | | PCR-F | Seq-F |
| ETFDHe7-R | ATCGCAAATAAGTTTTAGTCATAATGAACA | | PCR-R | Seq-R |
| ETFDHe8-71F | GACTTTTTTGTTTGCTTTTTTTTTTTTTTAG | | PCR-F | Seq-F |
| ETFDHe8-R | AAACCCTTATTTTATAACACTGATGGTAA | | PCR-R | Seq-R |
| ETFDHe9-F | TGCTTACATTTTAGCTTGATTTAATTTGA | | PCR-F | Seq-F |
| ETFDHe9-R | TCAATTCTGAATTTATAATACAGAAACAAGGA | | PCR-R | Seq-R |
| ETFDHe10-F | GGAGTATTCTGTTGTTCTTGTTTAATATGAA | | PCR-F | Seq-F |
| ETFDHe10-R | TAACCCAGCAATTTTCTTTTGTATACTT | | PCR-R | Seq-R |
| ETFDHe11-F | TTTGGGCAGTTTCGCACTTA | | PCR-F | Seq-F |
| ETFDHe11-R | TCTCTAATTAACAGATCCCATTCATGAA | | PCR-R | Seq-R |
| ETFDHe12-F | AAAATCATATTTTGTTAAGCATTTCCCT | | PCR-F | Seq-F |
| ETFDHe12-R | CACATTCCTAAAATGTTTAAAGCAAATATT | | PCR-R | Seq-R |
| ETFDHe13-F | TTCTGTGGCTACTCTTTCCTTAATTTT | | PCR-F | Seq-F |
| ETFDHe13-R | AGAGGTAGGAAGATGCTGCATTCT | | PCR-R | Seq-R |
| Subtotal | | 30 | | |
| | | | | |
| *HMGCS2* | | | | |
| Name | Sequence (5' To 3') | | | |
| HMGCS2-Ex1-S1 | GAGCCACGGTGAGCAGAG | | PCR-F | Seq-F |
| HMGCS2-Ex1-S2 | CTTGTCTAAGGCTGCGTGC | | PCR-R | Seq-R |
| HMGCS2-Ex2-S1 | GGGTCAATTCAATGAAAAGATAATAAC | | PCR-F | Seq-F |
| HMGCS2-Ex2-S2 | TGAGCAGAAGCCCATACGG | | PCR-R | Seq-R |
| HMGCS2-Ex2-S3 | TCCTGGCCCTGGAGGTC | | PCR-F | Seq-F |
| HMGCS2-Ex2-S4 | GGAGGCAGTACCACCGTAG | | PCR-R | Seq-R |
| HMGCS2-Ex2-S5 | GCGCATACAGCTCCCATGG | | PCR-F | Seq-F |
| HMGCS2-Ex2-S6 | GATGATAGCAGCAGCTGTGTG | | PCR-R | Seq-R |
| HMGCS2-Ex3-S2 | GTTTATCACTCTATATCCAGGACC | | PCR-F | Seq-F |
| HMGCS2-Ex3-S4 | AGCAATACCATCCTCAAACGC | | PCR-R | Seq-R |
| HMGCS2-Ex4-S1 | GACACAGATAATGACCAGAAACC | | PCR-F | Seq-F |
| HMGCS2-Ex4-S2 | CAAGGCAGGAGAGAAAAGACC | | PCR-R | Seq-R |
| HMGCS2-Ex5-S1 | AGTTCCCTGGGAGGCCTG | | PCR-F | Seq-F |
| HMGCS2-Ex5-S2 | GATTGCCATTTGTCCCCCAC | | PCR-R | Seq-R |
| HMGCS2-Ex6-S1 | GTAGGAAAAACATGCGCCCC | | PCR-F | Seq-F |
| HMGCS2-Ex6-S2 | CACTACACCAGGCCCCTTC | | PCR-R | Seq-R |
| HMGCS2-Ex7-F1 | AGAAGATCGGGATGATAGGC | | PCR-F | Seq-F |
| HMGCS2-Ex7-R1 | CTCAGTAATGGTGGGGAAG | | PCR-R | Seq-R |
| HMGCS2-Ex7-F2 | ACAGCCACTCTGCCCAAG | | PCR-F | Seq-F |
| HMGCS2-Ex7-R2 | CAAACTTACTCCATGCTTAAATCC | | PCR-R | Seq-R |
| HMGCS2-Ex8-S1 | GCCTGCATGTTTTGCTAACAAG | | PCR-F | Seq-F |
| HMGCS2-Ex8-S2 | AAATCCCTGGCTCCCAGC | | PCR-R | Seq-R |
| HMGCS2-Ex9-S1 | CCAGCCAAGAGAGCTTCAC | | PCR-F | Seq-F |
| HMGCS2-Ex9-S2 | TCCTGTCACCCCAATCCTC | | PCR-R | Seq-R |
| HMGCS2-Ex10-S1 | GGAGCCCCTCTGCACAG | | PCR-F | Seq-F |
| HMGCS2-Ex10-S2 | CTCCATCTTGCTCTTTCACAAAG | | PCR-R | Seq-R |
| HMGCS2-Ex10-S3 | CAGGGTGCCCAAGAGGAG | | PCR-F | Seq-F |
| HMGCS2-Ex10-S4 | ATGCACATTTCTGGAGTCCAG | | PCR-R | Seq-R |
| HMGCS2-Ex10-S5 | TGTCCTGGGCTTATGGTGC | | PCR-F | Seq-F |
| HMGCS2-Ex10-S6 | GCCCTCTGACCCACAAGG | | PCR-R | Seq-R |
| Subtotal | | 30 | | |
| | | | | |
| *MCAD* | | | | |
| Name | Sequence (5' To 3') | | | |
| MCAD-EX1-S1' | GAGGTGGAAACGCAGAAAAC | | PCR-F | Seq-F |
| MCAD-EX1-S2' | CTGCTCCGACACCACAATAC | | PCR-R | Seq-R |
| MCAD-Ex2-S1 | TCAAACCAGTTGCTGTACTCAC | | PCR-F | Seq-F |
| MCAD-Ex2-S2 | GTTCTACTCATTGAAAGACATTATTAAG | | PCR-R | Seq-R |
| MCAD-Ex3-S1 | AATCAAGGATTTAAGTCCCCAC | | PCR-F | Seq-F |
| MCAD-Ex3-S2 | ACCAAGTTCCCAGGCTCTTC | | PCR-R | Seq-R |
| MCAD-Ex4-S1 | GAATATGATAAAACTGGTGAAGTAGG | | PCR-F | Seq-F |
| MCAD-Ex4-S2 | AAACACTGTAGTCTGAGCAGG | | PCR-R | Seq-R |
| MCAD-Ex5-S1 | ACACACATTCCAGAGAACTGTG | | PCR-F | Seq-F |
| MCAD-Ex5-S2 | ACTGGGTCTGAGGAAAAGAATC | | PCR-R | Seq-R |
| MCAD-Ex6-S1 | GCCAGCCAGAACACATGTAG | | PCR-F | Seq-F |
| MCAD-Ex6-S2 | GTAGTAACTGACTTTCCTTTTTTTTTC | | PCR-R | Seq-R |
| MCAD-Ex7-S1 | CCAGTTCTTTGGACTTACCTG | | PCR-F | Seq-F |
| MCAD-Ex7-S2 | GGCATCCTTGACACATCATTG | | PCR-R | Seq-R |
| MCAD-Ex8-S1 | TTGAGTGGTTTCTTTGATTTTGTAAG | | PCR-F | Seq-F |
| MCAD-Ex8-S2 | CTTTAGGCCTTCCAGTTCCAC | | PCR-R | Seq-R |
| MCAD-Ex9-S1 | GGTAATAATAATTGGGATTGTTAAGAG | | PCR-F | Seq-F |
| MCAD-Ex9-S2 | CTGTTCACAAAATCTTCAGTAGTATATAGAC | | PCR-R | Seq-R |
| MCAD-Ex10-S1 | CAGACTACAGTTTGTTGATCCC | | PCR-F | Seq-F |
| MCAD-Ex10-S2 | GATATTAAAAAGAGAAACACACTGAAC | | PCR-R | Seq-R |
| MCAD-Ex11-S1 | CCTGTTCCCCACCAGTTTC | | PCR-F | Seq-F |
| MCAD-Ex11-S2 | TCTTTGAAACTCATCTTGATAAAACC | | PCR-R | Seq-R |
| MCAD-Ex12-S1 | CTGGGCAACATAGCAAGACC | | PCR-F | Seq-F |
| MCAD-Ex12-S3 | CTGAAATGGCAATGAAAGTTGAAC | | PCR-R | Seq-R |
| MCAD-Ex12-S2 | GATTTTGGCATCCCTCATTAG | | PCR-F | Seq-F |
| MCAD-Ex12-S4 | CAAAAACTGGCTCACAGCCATA | | PCR-R | Seq-R |
| MCAD-Ex13-S1 | AATCTGTAGAGGCTACAGACCC | | PCR-F | Seq-F |
| MCAD-Ex13-S2 | AACCTGCTTAAGAGACATAACAGAG | | PCR-R | Seq-R |
| Subtotal | | 28 | | |
| | | | | |
| *SLC22A5* | | | | |
| Name | Sequence (5' To 3') | | | |
| SCL22A5-F | AAATATGCAGCATTGCCC | | PCR-F | Seq-F |
| SCL22A5-R | CAGTGTTGAAAACGCACC | | PCR-R | Seq-R |
| SCL22A5-s1 | GCTTCTGAATAAACTCTGCACG | | | Seq-F |
| SCL22A5-s2 | CAGCGAGGTGATTCTAAAGC | | | Seq-R |
| SCL22A5-S3 | CAGTGACTTTCTCCTTACCTCC | | PCR-F | Seq-F |
| SCL22A5-S4 | CTGAGCAGGAAGAAGATGAG | | PCR-R | Seq-R |
| SCL22A5-s5 | GCGGCTGGCCTTACATAG | | | Seq-F |
| SCL22A5-s6 | GGGCCTCAGGTGCACTCCCG | | | Seq-R |
| SLC22A5ex1-F2 | CCCGGGCCTCAGGTGCACTC | | PCR-F | Seq-F |
| SLC22A5ex1-S4 | GCACCCGCCGCCGACCAGGCAAG | | PCR-R | Seq-R |
| SLC22A5ex1-S3 | GCGGCCCAGGCCCGCAACCTTCC | | | Seq-F |
| SLC22A5ex1-s1 | AGGCCGGTGAAGCCATTGGG | | | Seq-F |
| SLC22A5ex1-s2 | CATGCGGGACTACGACGAGGT | | | Seq-R |
| SLC22A5ex1-R | GTCCTCCGAGCCCTGGTCTC | | PCR-R | Seq-R |
| SLC22A5ex2-F | TCATTTTCCAGGATGCCTTTG | | PCR-F | Seq-F |
| SLC22A5ex2-R | CACGCTTCTTCCTCAGTGCTG ' | | PCR-R | Seq-R |
| SLC22A5ex3-F | GTGGAGCCCATTCCTGCTG | | PCR-F | Seq-F |
| SLC22A5ex3-R | TAGGTGATGGGATGATGGTGAAATAC | | PCR-R | Seq-R |
| SLC22A5ex4-F | AATAAGGAAGGAACCCAAATTAAACTG | | PCR-F | Seq-F |
| SLC22A5ex4-R | CAGACAGAAATCATCCTGCCAG | | PCR-R | Seq-R |
| SLC22A5ex5-F | CTGTGGGTCTGCTGTTGGC | | PCR-F | Seq-F |
| SLC22A5ex5-R | GGGTGCTGCTGCTCTCAAAT | | PCR-R | Seq-R |
| SLC22A5ex6-1-F | TGCTCTGAGTCTCTGACCACCTCT | | PCR-F | Seq-F |
| SLC22A5ex6-1-R | CCCAATGGCCACTTCAAGAA | | PCR-R | Seq-R |
| SLC22A5ex6-2-F | GTTGGGAAAGATGTGGATACTGC | | PCR-F | Seq-F |
| SLC22A5ex6-2-R | ATTAATTGAGACAGCCTGGTAGACAG | | PCR-R | Seq-R |
| SLC22A5ex6-s1 | GAGACAGCCTGGTAGACAGTAAGAGACTC | | | Seq-F |
| SLC22A5ex7-R | CCAGCTCACATTCAAGCCAGTT | | PCR-R | Seq-R |
| SLC22A5ex8-F | CCAAGTCTAACTGCAGCCCTG | | PCR-F | Seq-F |
| SLC22A5ex8-R | GCTCCTGAGACCTGGCCAG | | PCR-R | Seq-R |
| SLC22A5ex9-F | TGGAGACTGGGAGGCATCTTT | | PCR-F | Seq-F |
| SLC22ASex9-s1 | GGTTCTAGTGAAAAATTAACTGCTTTGG | | | Seq-F |
| SLC22ASex9- s | CATATGGCCCTAGAGCACCAC | | | Seq-R |
| SLC22A5ex9-s3 | GGTGATCTGCATCTGCTCATTC | | | Seq-F |
| SLC22A5ex9-s4 | GCACAAGGAGTTTGATTCTTACCTT | | | Seq-R |
| SLC22A5ex9-s5 | AAAACCCACATTACAGCTTTGAGAG | | | Seq-F |
| SLC22A5ex9-s6 | GCTCATAATAAATGCTCCATTGAATC | | | Seq-R |
| SLC22A5ex9-s7 | TGAGCTACTCTGAAAGACTATGAACACA | | | Seq-F |
| SLC22A5ex9-s8 | AGCCCTAGCCTCTAGCACTTCTC | | PCR-R | Seq-R |
| SLC22A5ex9-R | AAAGGCAGATGACAGTGTGGC | | | |
| Subtotal | | 40 | | |
| | | | | |
| *SLC25A20* | | | | |
| Name | Sequence (5' To 3') | | | |
| SLC25A20-F | GGGTTTCTCCATGTTGGTC | | PCR-F | Seq-F |
| SLC25A20-R | GGCCAGCAGGTTCTTGAG | | PCR-R | Seq-R |
| SLC25A20-s1 | CAGGCACAGACCATCTCAAG | | | Seq-F |
| SLC25A20-s2 | GCCTTCCCAGACTTCCATG | | | Seq-R |
| SLC25A20-s3 | TGTGTTGTACTACCAGGGGTG | | | Seq-F |
| SLC25A20-s4 | AAGGTGACAGGATTCAAGACC | | | Seq-R |
| SLC25A20ex1-F | GCTGAGAAGCCAGGACGGCCC | | PCR-F | Seq-F |
| SLC25A20ex1-R | CATGCTTTCCGCGCCCCG | | PCR-R | Seq-R |
| SLC25A20ex2-F | CTGACAGGCAGTTCTGATTCTGGTAG | | PCR-F | Seq-F |
| SLC25A20ex2-R | CAAATCAACCCCGTGAATGTGT | | PCR-R | Seq-R |
| SLC25A20ex3-F | AAAAGGTGGTGGTGTCTGTAAACA | | PCR-F | Seq-F |
| SLC25A20ex3-R | TCTTTATTTTAACCCATGTCACGCTA | | PCR-R | Seq-R |
| SLC25A20ex4-F | TCTGTCCTCGGTGGTTAGTCACAG | | PCR-F | Seq-F |
| SLC25A20ex4-R | GGACATAAACATGCACACGAAATTA | | PCR-R | Seq-R |
| SLC25A20ex5-F | GCTGGGTCTGTGACTCTGATGTTTCT | | PCR-F | Seq-F |
| SLC25A20ex5-R | GCAGCAGACCCACCTCAGGT | | PCR-R | Seq-R |
| SLC25A20ex6-F | GCTTTCAGATTCACCCACAGGAGAG | | PCR-F | Seq-F |
| SLC25A20ex6-R | CACCATGCCTGGCGAAGAGTT | | PCR-R | Seq-R |
| SLC25A20ex7-F | GCAGGATAGTCTGAATGCCACTCT | | PCR-F | Seq-F |
| SLC25A20ex7-R | CTTATGAGCTTTGCACCCCAG | | PCR-R | Seq-R |
| SLC25A20ex8-F | TTAACTGCTAGTTTCTCCTTCCTGAA | | PCR-F | Seq-F |
| SLC25A20ex8-R | AACAAGCAAAAGTCAAACCACATG | | PCR-R | Seq-R |
| SLC25A20ex9-F | GCAAAGCATAGTGCTTTGAATAGTCTATGAA | | PCR-F | Seq-F |
| SLC25A20ex9-s1 | GCAGGATGTCATTAAGGCAACAGTCTC | | | Seq-F |
| SLC25A20ex9- s2 | TCTGGATGCTGGAAGCTGTCGTT | | | Seq-R |
| SLC25A20ex9-R | TGAGAACCTGAGATTCTCTCTTTAATGAGG | | PCR-R | Seq-R |
| Subtotal | | 39 | | |
| *HMGCL* | | | | |
| Name | Sequence (5' To 3') | | | |
| HMGCL-F | GCATTTTCTTTGCCTCAGG | | PCR-F | Seq-F |
| HMGCL-R | ACCCTGACAGTCAGAGTGC | | PCR-R | Seq-R |
| HMGCL-s1 | CTCTAAACACAAGAAGCCACAG | | | Seq-F |
| HMGCL-s2 | TGTAATCCTAGCACTTTGGGAG | | | Seq-R |
| HMGCL-s3 | GTTTCGCCATGTTAGCCAG | | | Seq-F |
| HMGCL-s4 | CGCCTGTAATCCCAGCTAC | | | Seq-R |
| HMGCLex1-F | GACGAGCCATCGGTCACG | | PCR-F | Seq-F |
| HMGCLex1-R | CCCAACCCTGACAGTCAGAGT | | PCR-R | Seq-R |
| HMGCLex2-F | GGAATTGTGGCAATCTCTTCC | | PCR-F | Seq-F |
| HMGCLex2-R | GCCATTGCACCTATCACACGTA | | PCR-R | Seq-R |
| HMGCLex3-F | CATTTTGAGGCTGTTTTGTTACTAACT | | PCR-F | Seq-F |
| HMGCLex3-R | CTTGCTTCAAAGGCAAATGC | | PCR-R | Seq-R |
| HMGCLex4-F | ACTCTCTGTCTGCTCTTGGTGATG | | PCR-F | Seq-F |
| HMGCLex4-R | GCGCCAAGACAAGGCAGG | | PCR-R | Seq-R |
| HMGCLex5-F | TGGATGTTGGCAGGTTGCT | | PCR-F | Seq-F |
| HMGCLex5-R | TTTGTGGCAGGAGGACCA | | PCR-R | Seq-R |
| HMGCLex6-F | AGCCAAAAAGCTCAGGACAC | | PCR-F | Seq-F |
| HMGCLex6-R | ACCTATGCGCTCAGG | | PCR-R | Seq-R |
| HMGCLex7-F | AGTGCATGGATCCCCTGG | | PCR-F | Seq-F |
| HMGCLex7-S1 | CACCATTGGTGTGGGCACCC | | | Seq-F |
| HMGCLex7-S2 | GGTGTCATGGCAGTGGACAG | | | Seq-R |
| HMGCLex7-R | TGCTGAAAGAATAAATGATAACAAGC | | PCR-F | Seq-F |
| HMGCLex8-F | GGCCTCTTGTCCCCAGCAA | | PCR-F | Seq-F |
| HMGCLex8-R | CCCATCCACTTTTGTTCTCAGC | | PCR-R | Seq-R |
| HMGCLex9-F | TGATGTTTTCCCTGGTGTTGA | | PCR-F | Seq-F |
| HMGCLex9-s1 | CCACACGTCCTCAGGCATT | | | Seq-F |
| HMGCLex9-s2 | ACCTCCTGCCCAGACCTG | | | Seq-R |
| HMGCLex9-R | TCTCCATTTTCCTCTCAGAGTGC | | PCR-R | Seq-R |
| Subtotal | | 28 | | |
| | | | | |
| *ACAT1* | | | | |
| Name | Sequence (5' To 3') | | | |
| ACAT1ex1-F | TGAGAGGCGACTATTGGAGGA | | PCR-F | Seq-F |
| ACAT1ex1-R | AAGCTTCCGAGGCGGG | | PCR-R | Seq-R |
| ACAT1ex2-F | TGCAGGAAGAAACCACTATAACCTT | | PCR-F | Seq-F |
| ACAT1ex2-R | TTTATTGACATTTCCTTTAAATTCTGGA | | PCR-R | Seq-R |
| ACAT1ex3-F | TTAATGAAATACGATTTGGGTAAAATACCTATAA | | PCR-F | Seq-F |
| ACAT1ex3-R2 | TTCCATGGAATCTTTTTGCTCCC | | PCR-R | Seq-R |
| ACAT1ex4-F | ATTCCTATTGTGTAATGTCACCTACCTTATT | | PCR-F | Seq-F |
| ACAT1ex4-R | CGGCCCCTTTTACACATTTTAAGTA | | PCR-R | Seq-R |
| ACAT1ex5-F | CAGTTGTGTTTGAGTATCGGTTTTTCA | | PCR-F | Seq-F |
| ACAT1ex5-R | CTAGATTTTTGTGTCTTTTAGCCCTAGGTAT | | PCR-R | Seq-R |
| ACAT1ex7-F | GTAGCTGTATAAAGGGTGTCATGGGTT | | PCR-F | Seq-F |
| ACAT1ex7-R | GCCCATCTTTCAGTTCAGTTTGTAAAGT | | PCR-R | Seq-R |
| ACAT1ex8-F | GGGCAGCTGTGCTGAGAATACAG | | PCR-F | Seq-F |
| ACAT1ex8-R | GTTGAAATGAACCAAGATAATGTCC | | PCR-R | Seq-R |
| ACAT1ex8-S1 | CACTATAAGTTAGGCAAAGTTAATAG | | | Seq-F |
| ACAT1ex9-F | TGAATGACTACTTGTTTTGAGCGATT | | PCR-F | Seq-F |
| ACAT1ex9-R | GGCAATTCTAAGAACTAGATATGGTATACTGG | | PCR-R | Seq-R |
| ACAT1ex9-F2 | CATCTTGTACAACAGTTGCTTGC | | PCR-F | Seq-F |
| ACAT1ex9-R2 | AGATGTTTTCTAGAATTTTTCAAAATCC | | PCR-R | Seq-R |
| ACAT1ex10-F2 | TTAAACATTTAGCAGCCCAGGCAATAG | | PCR-F | Seq-F |
| ACATlex10-s1 | GGCAAGAATAGTAGGTAAGGCC | | | Seq-F |
| ACAT1ex10-s2 | CTATATTGCCCAGGCTGATTTCG | | | Seq-R |
| ACAT1ex10-R | TAGAGACGAAGTCTCACTATATTGCCC | | PCR-F | Seq-F |
| ACAT1ex11-F | TAGGAAATGTGCATTTAATGGGC | | PCR-R | Seq-R |
| ACAT1ex11-R | AACATTTAAAATCTTAAAAACTGCAACC | | PCR-F | Seq-F |
| ACAT1ex12-F2 | CTTATGTCAAACTGTAGTATTTCAAGG | | PCR-R | Seq-R |
| ACATlex12-R2 | GTTTAGCTAGAAACACAAAGGCAGCTC | | PCR-F | Seq-F |
| ACAT1ex13-F | CATTTTGGTTAGTCATAAATTCTGTACTTCATTA | | PCR-R | Seq-R |
| ACATlex13-R | GACTCAGAATGCAAAAATGTATCAAACTTT | | PCR-F | Seq-F |
| Subtotal | | 29 | | |
| | | | | |
| *CPT1A* | | | | |
| Name | Sequence (5' To 3') | | | |
| CPT1A-F | ACACAGACACCAGAGGCAG | | PCR-F | Seq-F |
| CPT1A-R | ATGAAGTCCAGAGAGATAGATTTG | | PCR-R | Seq-R |
| CPT1A-s1 | GCCAGAGTTGAGGTACCAC | | | Seq-F |
| CPT1A-s2 | GCTTAGAGCCCCAAGATGATC | | | Seq-R |
| CPT1Aex1-F2 | TCAGCCAATCCGCTGCTGCCG | | PCR-F | Seq-F |
| CPTIAex1-R2 | CGGTCCGGTTCCCGGCAGC | | PCR-R | Seq-R |
| CPT1Aex2-F | TGAGGGTTTTTTAGTGTCTGGTCACA | | PCR-F | Seq-F |
| CPT1Aex2-R | GCAGCCAGAGCCCCGTTC | | PCR-R | Seq-R |
| CPT1Aex3-F | CCTGTGGGTTGTGGGAGGAC | | PCR-F | Seq-F |
| CPT1Aex3-R | CACATTTGAAGAGACATAAAAACAGCAA | | PCR-R | Seq-R |
| CPT1Aex4-F | GGCACCGTGTGGCCTCAC | | PCR-F | Seq-F |
| CPT1Aex4-R | GCCCAAACTGTCCACCCCC | | PCR-R | Seq-R |
| CPT1Aex5-F | CCTTCAGAGTAGTATGGCAGTAGTCTGAAA | | PCR-F | Seq-F |
| CPT1Aex5-R | GACCTAGTTCATCATAATTAAGACCTAAGCCT | | PCR-R | Seq-R |
| CPT1Aex6-F | TAAGGCACAAGTGAGGCAAACATAG | | PCR-F | Seq-F |
| CPT1Aex6-R | GCCCCTCAAAATTAGCGTCTTCA | | PCR-R | Seq-R |
| CPT1Aex7-F | CCGCGGCTCTGGGCT | | PCR-F | Seq-F |
| CPT1Aex7-R | TGTGAAGACGCCACCTCTGTG | | PCR-R | Seq-R |
| CPT1Aex8-F | TGCAGGATGTGCTGTGATTATTTAA | | PCR-F | Seq-F |
| CPT1Aex8-R | ATGTGCAATATGTCAATTATACCTCTGTAAAG | | PCR-R | Seq-R |
| CPT1Aex9-F | TTTCTTCTGTGGGATTTTCGTTG | | PCR-F | Seq-F |
| CPT1Aex9-R | CCTTTGACACTTCCTAATTCTGAGCAT | | PCR-R | Seq-R |
| CPT1Aex10-F | GGGACTCTTGGTTTTGTGTCTCC | | PCR-F | Seq-F |
| CPT1Aex10-R | CCCAGTGAGGAAGAGCGCC | | PCR-R | Seq-R |
| CPT1Aex11-F | CCATCGCTGCAGGTCGGG | | PCR-F | Seq-F |
| CPT1Aex11-R | GGGTATTTCTTCCAAGCTCATGGG | | PCR-R | Seq-R |
| CPT1Aex12-F | CCTCATTGTTGCCTCTTTAAACACTT | | PCR-F | Seq-F |
| CPT1Aex12-R | CACTGCGCCTGGCCAG | | PCR-R | Seq-R |
| CPT1Aex13-F | CTTCATGTTGGAGGTTAATGTGTTTTA | | PCR-F | Seq-F |
| CPT1Aex13-R | ACGGTTGGAAAATTCATCTGTAAGAC | | PCR-R | Seq-R |
| CPT1Aex14-F | GGTGTGCGTCAGTACTTTTCTCCC | | PCR-F | Seq-F |
| CPT1Aex14-R | TGGGTGAACAGTCTTGGGCA | | PCR-R | Seq-R |
| CPT1Aex15-F | AGACATATCAGCATGCCCTGCC | | PCR-F | Seq-F |
| CPT1Aex15-R | GGCCTCCAGAAGCCTTTTAGG | | PCR-R | Seq-R |
| CPT1Aexl6-F | ACTGCTCAACTTGAGACATGCAAC | | PCR-F | Seq-F |
| CPT1Aex16-R | AAAATATGTTCATGCAAGGCTGTAGTAA | | PCR-R | Seq-R |
| CPT1Aex17-F | TGAGGGACGGTAGAGGAGGG | | PCR-F | Seq-F |
| CPT1Aex17-R | ATCACACCCCATTACCCATCC | | PCR-R | Seq-R |
| CPT1Aex18-F | GTCCCTTTTTCACGTTGTCTCATT | | PCR-F | Seq-F |
| CPT1Aex18-R | TTTCTGTCAAATTAAAGATGTGTTTCCTTA | | PCR-R | Seq-R |
| CPT1Aex19-F | AACATTGGGTCAGATTGCAAATG | | PCR-F | Seq-F |
| CPT1Aex19-R | AGTGTTTCATCCCGAGCTAAGGTC | | PCR-R | Seq-R |
| Subtotal | | 42 | | |
| | | | | |
| *HADHA* | | | | |
| Name | Sequence (5' To 3') | | | |
| HADHAex1-F | GTCAGGCAGTGCTCCAGGC | | PCR-F | Seq-F |
| HADHAex1-R | GAAAGGGAGACGCGGCTC | | PCR-R | Seq-R |
| HADHAex2-F | TGTTGTTGTTATTATTGTAAATTACAGCTTTG | | PCR-F | Seq-F |
| HADHAex2-R | CATAGTTAATATGGGTTCTGGCTAAAAAGA | | PCR-R | Seq-R |
| HADHAex3-F | ACAGGGTCTTCTGCTTTGCTGA | | PCR-F | Seq-F |
| HADHAex3-R | CTGCCAGATTGGTAGATTTGGG | | PCR-R | Seq-R |
| HADHAex4-F | GCTACTGACTTGCTGGGAGAATGA | | PCR-F | Seq-F |
| HADHAex4-R | TGTGCCCAGCACTTCTACTTTCTT | | PCR-R | Seq-R |
| HADHAex4-S1 | ATTTGATGGTGGTACGTACATCC | | | Seq-F |
| HADHAex5-F2 | GTGTATAGTGAATAAGTATTGAACC | | PCR-F | Seq-F |
| HADHAex5-R | GCCTAAGGGTTTACAGCTGATGAA | | PCR-R | Seq-R |
| HADHAex6-F | CCCTTATCCATCTCAAGTTGCTACTAA | | PCR-F | Seq-F |
| HADHAex6-R | TGCCCATATTATGCATTTTATACAAGAAA | | PCR-R | Seq-R |
| HADHAex7-F | ATTATCACCCTGATTTTTTTCCCC | | PCR-F | Seq-F |
| HADHAex7-R | GCCTGGCCTAAGAGGTTAACTCTT | | PCR-R | Seq-R |
| HADHAex8-F | TCTGGGTTTTTCACTACCTGGGA | | PCR-F | Seq-F |
| HADHAex8-R | ACTTCCTCATTTTGAATCTACAGCAAA | | PCR-R | Seq-R |
| HADHAex9-F | CATAAACTAACAAGGGAATCTAGGCTCTT | | PCR-F | Seq-F |
| HADHAex9-R | GAATGGCAATAAGGAGGAGTGATCTA | | PCR-R | Seq-R |
| HADHAex10-F | TTTATTCAGCATTATTGTACATCTCGCC | | PCR-F | Seq-F |
| HADHAex10-R | TTTCAGCTTTATACAGAGGATTGGATCT | | PCR-R | Seq-R |
| HADHAex11-F | CTGAACTGAAGGAATCCATATAACCTAGA | | PCR-F | Seq-F |
| HADHAex11-R | TGTAGATCTTCAAAGCCTCTGTGAACT | | PCR-R | Seq-R |
| HADHAex12-F | GGCAATCTTCCACTTTGACTGAA | | PCR-F | Seq-F |
| HADHAex12-R | CCAAGCCACCACAAAAAACAACTA | | PCR-R | Seq-R |
| HADHAex13-F | TGGCCCATGGACATTCAATAA | | PCR-F | Seq-F |
| HADHAex13-R | CATGAGTTTCCTATAGCTCCTTTAAAAAAAA | | PCR-R | Seq-R |
| HADHAex13-S1 | TATAGCTCCTTTAAAAAAAAAATCTCTGTG | | | Seq-F |
| HADHAex14-F | CTCATTAGCTTTGATGTGGCCTCT | | PCR-F | Seq-F |
| HADHAex14-R | CTTCTGTACTCAAGCTGTAAGCCTTTATC | | PCR-R | Seq-R |
| HADHAex15-1-F | CTTTCTTTCTAGTGTTTTCCTAGCAGAGA | | PCR-F | Seq-F |
| HADHAex15-1-R | ATCATATCAAAGTCTGGTCATTTGCC | | PCR-R | Seq-R |
| HADHAex15-1-S1 | ATAAACAAGCCTGGAGGTAAAAGG | | | Seq-F |
| HADHAex15-2-F | TGACCCTGGGAAAGGTAGAGTG | | PCR-F | Seq-F |
| HADHAex15-2-R | TGTATCAGAAGGAAGCTTGGTCCT | | PCR-R | Seq-R |
| HADHAex15-3-F | CTTTGGGGGGATTTGGGCTGCC | | PCR-F | Seq-F |
| HADHAex15-3-R | GCTTCTGTAACTCTTTGGTCTCAGG | | PCR-R | Seq-R |
| HADHAex16-F | CCAGGCCAGGGTTCTGAGA | | PCR-F | Seq-F |
| HADHAex16-R | GCACATGTATCCCAGGACTTAAAGTATTA | | PCR-R | Seq-R |
| HADHAex17-F | TCCCGACTTCCATTCTGCATCT | | PCR-F | Seq-F |
| HADHAex17-R | GACTTCGTTGAAGGAGACGCAA | | PCR-R | Seq-R |
| HADHAex18-1-F | GGAAGGAAGCTCACTTTAGCCTG | | PCR-F | Seq-F |
| HADHAex18-1-R | CCGGAGTTTGTCTTCTCGTTACTC | | PCR-R | Seq-R |
| HADHAex18-2-F | GAAGTTCTACCAGTGAGCAGGCC | | PCR-F | Seq-F |
| HADHAex18-2-R | GAGGTGGCTTCAGATGGCTCTT | | PCR-R | Seq-R |
| HADHAexl8-3-F | TCTCTCCCTCCTGGTGAAGTGTG | | PCR-F | Seq-F |
| HADHAex18-3-R | TCCCACTTTCTCTCATCCCAGTC | | PCR-R | Seq-R |
| HADHAex18-4-F | GCATCTTTGCCCTTCTGGTTTAA | | PCR-F | Seq-F |
| HADHAex18-4-R | CTCTGCTTAGCACCAACGTTCC | | PCR-R | Seq-R |
| Subtotal | | 49 | | |
| | | | | |
| *HADHB* | | | | |
| Name | Sequence (5' To 3') | | | |
| HADHAB-F | TCAACTCCCACTTTCCCAG | | PCR-F | Seq-F |
| HADHAB-R | GTCCCGGCAGGAGTTCTG | | PCR-R | Seq-R |
| HADHAB-S1 | TCTCGGAGCAAGGTTCAG | | | Seq-F |
| HADHAB-S2 | AGCACTGCCTGACTAAAACC | | | Seq-R |
| HADHBex1-F | GCCGGAGGGCAGCCCT | | PCR-F | Seq-F |
| HADHBex1-R | CGGCGACAGTTGCGGG | | PCR-R | Seq-R |
| HADHBex2-F | GCCATGGATGAGGTTATCAGAGTT | | PCR-F | Seq-F |
| HADHBex2-R | CCATTCAGTATCTGTGCGAGATGT | | PCR-R | Seq-R |
| HADHBex3-F | TTTTTTTATTTTTAGAGATTGGATTTGGAT | | PCR-F | Seq-F |
| HADHBex3-R | TTAGTTTTGGTTTTTATTCAGAGTTCACAA | | PCR-R | Seq-R |
| HADHBex5-F | GGTGACAGAGCGAGACTCCATCT | | PCR-F | Seq-F |
| HADHBex5-R | AATTTCATGGGACTGCTATCCAAA | | PCR-R | Seq-R |
| HADHBex6-F | GATCTCTGGGTATTTCAAATAGAATGAAAA | | PCR-F | Seq-F |
| HADHBex6-R | TGCTTAAATTTGTATAGTACAGATGGCTAAAA | | PCR-R | Seq-R |
| HADHBex7-F | TGATCTCTGGCACTGCCTTGAT | | PCR-F | Seq-F |
| HADHBex7-R | AGGCTTTTGTAAAGTAACTTCTATTAATTGAAAT | | PCR-R | Seq-R |
| HADHBex8-F | GATTAGTGCTCAAAGCATCATTTACATG | | PCR-F | Seq-F |
| HADHBex8-R | GAGGTTCTGTGTTAAAAAAAAAAAAAAAAA | | PCR-R | Seq-R |
| HADHBex9-F | AGAAGCTTAAATTGGAATGGTATGTTATT | | PCR-F | Seq-F |
| HADHBex9-R | ACAGCATAGCAGAGTCCACACTG | | PCR-R | Seq-R |
| HADHBex10-F | AATCCTAGGTGTTAGCATAACACCG | | PCR-F | Seq-F |
| HADHBex10-R | CACTCGGGATGTTTTTGAGTTTT | | PCR-R | Seq-R |
| HADHBex11-F | GGTAAGGTTTATATTTCATTTGTTTTTGTAATTT | | PCR-F | Seq-F |
| HADHBex11-R | TCATAACACACACAAAAAGTTATATTAGGCTT | | PCR-R | Seq-R |
| HADHBex12-F | GATATATAAGATGGCTGGAGAAAGACCTC | | PCR-F | Seq-F |
| HADHBex12-R | GACACATACATAAAATCCCTAGGTAAAAAAAT | | PCR-R | Seq-R |
| HADHBex13-F | CCGAAGGCATATTTGAGGTAAAGTAA | | PCR-F | Seq-F |
| HADHBex13-R | TCAGGGCTTAATATACTCTCAAACCAGT | | PCR-R | Seq-R |
| HADHBex14-F | CATTAGAGTACCTATGTAAAACTCAGTTTGGG | | PCR-F | Seq-F |
| HADHBex14-R | TCATAAACCATGCCTTGCTCTCTCC | | PCR-R | Seq-R |
| HADHBex15-F | AACCATTCCTATAAGAAAGCGTAGAGG | | PCR-F | Seq-F |
| HADHBex15-R | TCAAAAGAAAGAGAAGCTTTCCCTT | | PCR-R | Seq-R |
| HADHBex16-F | AGTACTAAGAGCCTAGCTTGATTCTGATCTTAAG | | PCR-F | Seq-F |
| HADHBex16-R | AAAAATATTGCTTTAAGAAGGTTTCAAACTAA | | PCR-R | Seq-R |
| HADHBex17-F | CTGTCCCTGTACTAGGTGGATTCA | | PCR-F | Seq-F |
| HADHBex17-R | TCTTTATCTTCCCAGCAGCCTC | | PCR-R | Seq-R |
| Subtotal | | 36 | | |
| | | | | |
| *VLCAD* | | | | |
| Name | Sequence (5' To 3') | | | |
| VLCAD-F | TTTCCATGTCTCTGCCTCTG | | PCR-F | Seq-F |
| VLCAD-R | GGCACCAAGAGGGAGATC | | PCR-R | Seq-R |
| VLCAD-S1 | TTCTGTCCCATCCTATCCC | | | Seq-F |
| VLCAD-S2 | GGTACCTGGCACCAAGAG | | | Seq-R |
| VLCADex1-F2 | CACTCCAGGGGGCACCTGTGG | | PCR-F | Seq-F |
| VLCADex1-R | CCCCAACATGGACTGTCTCTGTAT | | PCR-R | Seq-R |
| VLCADex1-S1 | CACTCCAGGGGGCACCTGTGG | | | Seq-F |
| VLCADex2-F | CGAGGGACGCTTGGAGATCC | | PCR-F | Seq-F |
| VLCADex2-S2 | CTCGGATGGCCGCGAGCTTG | | PCR-R | Seq-R |
| VLCADex2-S1 | CACCCCCGGCATAGGGCCG | | | Seq-F |
| VLCADex2-R | GATCCCTGCCGATGGCGGGC | | PCR-R | Seq-R |
| VLCADex3-F2 | GTCACCGCTTCGCGCCGCCTC | | PCR-F | Seq-F |
| VLCADex3-R2 | GGTGAGCTGGCCTTTGAACATTCCC | | PCR-R | Seq-R |
| VLCADex4-F | TGACCAGGAAAAAACCGGC | | PCR-F | Seq-F |
| VLCADex4-R | AGCTCTTTAAGAAACTGTGTCTGCTCT | | PCR-R | Seq-R |
| VLCADex5-F | ATACCCGTCCGGTAAGGGAAG | | PCR-F | Seq-F |
| VLCADex5-R | CATTCTTGGCGGGATCGTT | | PCR-R | Seq-R |
| VLCADex6-F | CTGGTGAGGTGTTTGGAGATGTT | | PCR-F | Seq-F |
| VLCADex6-R | CATACTGGGATGTGGCGATAGG | | PCR-R | Seq-R |
| VLCADex7-F | CTCCTGTTAAGGTCAGGTCCCC | | PCR-F | Seq-F |
| VLCADex7-R | TCTCCTAGGGTTGCCTCACCAG | | PCR-R | Seq-R |
| VLCADex8-F | TGGATACTCCCAGGTGTTAAGGG | | PCR-F | Seq-F |
| VLCADex8-S1 | GAAGAATGGGATATTCAGGGCG | | | Seq-F |
| VLCADex8-S2 | CCTTCTCCTCCGCCCAATT | | | Seq-R |
| VLCADex8-S3 | GGGTGACTTGCAACAGCCA | | | Seq-F |
| VLCADex8-S4 | GCGAAGGAGCAGTTTTTCCCC | | | Seq-R |
| VLCADex8-S5 | CAGGGACACATGGCAAGGG | | | Seq-F |
| VLCADex8-S6 | CCCTCTGGCGCAAGCC | | | Seq-R |
| VLCADex8-R | GAGACCCACTGCCCCAGGT | | PCR-R | Seq-R |
| VLCADex9-F | GGTGATGAGGCCAAGTCTGACA | | PCR-F | Seq-F |
| VLCADex9-R | GAACAGATGAGACTGGTTTTGGG | | PCR-R | Seq-R |
| VLCADex10-F | AGCAAAATCTTTGGCTCGGTGAG | | PCR-F | Seq-F |
| VLCADex10-R | CGAAGATCTCGGAGCACACGCT | | PCR-R | Seq-R |
| VLCADex11-F | AAGGTACAGGACGGTCTTCTGCA | | PCR-F | Seq-F |
| VLCADex11-R | GCTCCTTTCCTTTGTCCTATGGG | | PCR-R | Seq-R |
| VLCADex12-F | TGGTAAGACAGAGAATTGGGTGG | | PCR-F | Seq-F |
| VLCADex12-R | CCCGCCTGAGAGGAAGAGG | | PCR-R | Seq-R |
| VLCADex13-F | GAGGCAGGCAAACAGCTGAG | | PCR-F | Seq-F |
| VLCADex13-R | CACAGTGGCAAACTGCTCCA | | PCR-R | Seq-R |
| VLCADex14-F | CGGAGTGGCGAGCTGGTAAGT | | PCR-F | Seq-F |
| VLCADex14-R | TCTGCCAGCCGCTGCA | | PCR-R | Seq-R |
| VLCADex15-F | CTCCTTGAGACTAATGCCCCCA | | PCR-F | Seq-F |
| VLCADex15-R | GCAGGAAGCAGGAGATGAAAATA | | PCR-R | Seq-R |
| VLCADex16-F | AGGGAATGCCTGAGCCG | | PCR-F | Seq-F |
| VLCADex16-R | ATGCCCTCTCGGATCCG | | PCR-R | Seq-R |
| VLCADex17-F | TGACACCTGGTGTATCGAGGTGA | | PCR-F | Seq-F |
| VLCADex17-R | CAATAGAGGCAAACTCCCCACG | | PCR-R | Seq-R |
| Subtotal | | 47 | | |
| | | | | |
| *LCAD* | | | | |
| Name | Sequence (5' To 3') | | | |
| LCAD-F | TGGCTAACACGGTGAAATC | | PCR-F | Seq-F |
| LCAD-R | GGAGGACGATCAGCTGAG | | PCR-R | Seq-R |
| LCADex1-F | GCAAAGGCAGCAGTTTCGCT | | PCR-F | Seq-F |
| LCADex1-R | GAGTGACTCGCTGCCCCA | | PCR-R | Seq-R |
| LCADex2-F | CAGTGGCACTAATTTGGATATGAAGA | | PCR-F | Seq-F |
| LCADex2-R | AAAGCCCTGAACATACTATGTTTTCTG | | PCR-R | Seq-R |
| LCADex3-F | CATAAGTTCAAAGTTGTGGCCATT | | PCR-F | Seq-F |
| LCADex3-R | CAAGAGTGCATGAGCGGTATAATC | | PCR-R | Seq-R |
| LCADex4-F | ACTACCAAACAATTTGATAGGACCTTAAT | | PCR-F | Seq-F |
| LCADex4-R | CCTTCCTCCTAGCATATAGCTCAGTC | | PCR-R | Seq-R |
| LCADex5-F | GCAATTTTATAATGGTGTAGCCAGTTTG | | PCR-F | Seq-F |
| LCADex5-R | GAGTCCCTAGATTTAATAGAATTAATGAGTTGTC | | PCR-R | Seq-R |
| LCADex6-F | TTTCTCCCAATCATAACTTGCAC | | PCR-F | Seq-F |
| LCADex6-R | CATGTAATCCTCTATGTAAGTCTCCTATCTGA | | PCR-R | Seq-R |
| LCADex7-F | CAATTTGTGTTTTGTTTTTTTGGAAA | | PCR-F | Seq-F |
| LCADex7-R | CACAAGTTTTGCATGTATAAATTTGAATG | | PCR-R | Seq-R |
| ICADex8-F | AATTGTGTGAAAATCACTTGCATAATT | | PCR-F | Seq-F |
| LCADex8-R | CAAACTTCTATTTACCCCAGGCC | | PCR-R | Seq-R |
| LCADex9-F | TGATATGTTAAGTGGGTTGAAGTTTATATTTCTA | | PCR-F | Seq-F |
| LCADex9-R | GCTGAGCTTAAAATTGGCAGAATTC | | PCR-R | Seq-R |
| LCADex10-F | CCGTTCTAATTGCTTAATAAATGTGCA | | PCR-F | Seq-F |
| LCADex10-R | CTTTCCCTCTACATTTGATGTACAAAGTAAA | | PCR-R | Seq-R |
| LCADex11-F | TTGAATAGTTTATGAGTTACCTGGTAAGAC | | PCR-F | Seq-F |
| LCADex11-R | TTTTATTTCCTTCTCTATAGAGAGAGC | | PCR-R | Seq-R |
| Subtotal | | 24 | | |
| | | | | |

### Results

### 1. Detection of genetic polymorphism

Genetic polymorphisms (single nucleotide polymorphism: SNP, or insertion, deletion) at 45 positions were detected in the specimens from the patients with influenza-associated encephalopathy and the patient suspected to have influenza-associated encephalopathy due to suspected CPTII deficiency as shown in FIG. 4.

### 1-1. Genetic polymorphisms where single nucleotide polymorphism was observed at significantly higher frequency in the patients compared with healthy subject control by Fisher's two-sided test (p<0.05)

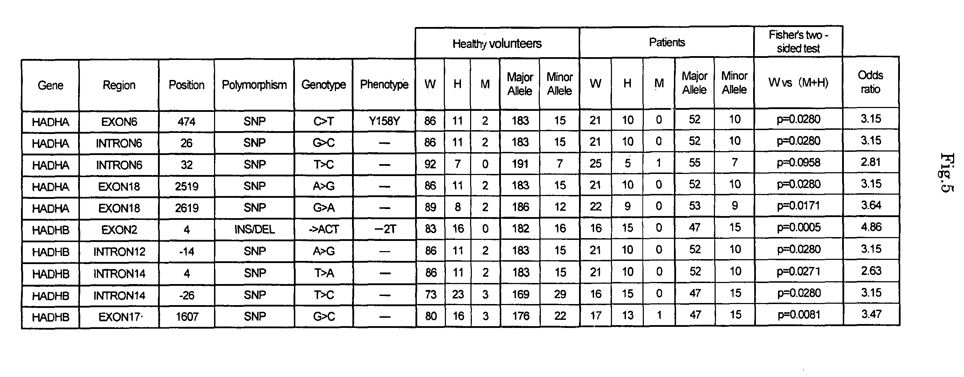

The genetic polymorphisms observed at higher frequency in the patients were detected in HADHA the 6th exon region (EXON 6), HADHA the 6th intron region (IBTRON 6), HADHA the 18th exon region (EXON 18), HADHB the 2nd exon region (EXON 2), HADHB the 12th intron region (INTRON 12), HADHB the 14th intron region (INTRON 14), and HADHB the 17th exon region (EXON 17). Those were listed in FIG. 5.

The positions of the genetic polymorphisms corresponding to the exon regions are described by making the position of A in an initiation codon ATG a base number 1. The base number corresponding to the intron region was described by the position upstream (represented by -) or downstream (represented by +) from the end of the position corresponding to the exon region, and a nucleic acid number in NCBI database.

SNP having the genotypes of C/C, C/T and T/T at base number 474 in HADHA was detected. This SNP was cSNP which did not affect the phenotype. Those having a minor allele T (healthy subjects 11+2=13, patients 10+0=10) were present at a significantly (p=0.028) higher frequency in the patients compared with those not having the minor allele T (healthy subjects 86, patients 21), and its odds ratio was 3.15 times. This SNP was a novel SNP not reported conventionally in HGMD database and dbSNPs.

SNP having the genotypes of G/G, G/C and C/C was detected at position +26 (NT_022184.14 Number 5270935) downstream of the exon 6, in the 6th intron of HADHA. This SNP was an intron SNP. Those having a minor allele C (healthy subjects 11+2=13, patients 10+0=10) were present at a significantly (p=0.028) higher frequency in the patients compared with those not having the minor allele C (healthy subjects 86, patients 21), and its odds ratio was 3.15 times. This SNP was a novel SNP not reported conventionally in HGMD database and dbSNPs.

SNP having the genotypes of T/T, T/C and C/C was detected at position +32 (NT_022184.14 Number 5270929) downstream of the exon 6x, in the 6th intron of HADHA. This SNP was the intron SNP. Those having a minor allele C (healthy subjects 5+1=6, patients 7+0=7) were present at not significantly (p=0.0958) but higher frequency in the patients compared with those not having the minor allele C (healthy subjects 82, patients 25), and its odds ratio was 2.81 times.

SNP having the genotypes of A/A, A/G and G/G at base number 2519 in HADHA was detected. This SNP was cSNP which did not affect the phenotype. Those having a minor allele G (healthy subjects 11+2=13, patients 10+0=10) were present at a significantly higher frequency (p=0.0280) in the patients compared with those not having the minor allele G (healthy subjects 86, patients 21), and its odds ratio was 3.15 times.

SNP having the genotypes of G/G, G/A and A/A at base number 2619 in HADHA was detected. This SNP was cSNP which did not affect the phenotype. Those having a minor allele A (healthy subjects 8+3=11, patients 9+0=9) were present at a significantly higher frequency (p=0.0171) in the patients compared with those not having the minor allele A (healthy subjects 89, patients 22), and its odds ratio was 3.64 times.

The genetic polymorphism having the genotypes of ACT/ACT or -/ACT (insertion) was detected at base number 4 in HADHB. This polymorphism generates the phenotype where threonine (T) is inserted at position 2 in the amino acid sequence in the case of the allele having ACT. Those having the allele with ACT insertion (healthy subjects 16+0=16, patients 10+0=10) were present at a significantly (p=0.0005) higher frequency in the patients compared with those not having the allele with ACT insertion (healthy subjects 83, patients 16), and its odds ratio was 4.86 times.

SNP having the genotypes of A/A, A/G and G/G was detected at position -14 (NT_022184.14 Number 532179) upstream of the exon 13, in the 12th intron of HADHB. This SNP was the intron SNP. Those having a minor allele G (healthy subjects 11+2=13, patients 10+0=10) were present at a significantly (p=0.0280) higher frequency in the patients compared with those not having the minor allele G (healthy subjects 86, patients 21), and its odds ratio was 3.15 times. This SNP was a novel SNP not reported conventionally in HGMD database and dbSNPs.

SNP having the genotypes of T/T, T/C and C/C was detected at position +4 (NT_022184.14 Number 5323009) downstream of the exon 14, in the 14th intron of HADHB. This SNP was the intron SNP. Those having a minor allele C (healthy subjects 11+2=13, patients 10+0=10) were present at a significantly (p=0.0280) higher frequency in the patients compared with those not having the minor allele C (healthy subjects 86, patients 21), and its odds ratio was 3.15 times.

SNP having the genotypes of T/T, T/A and A/A was detected at position -26 (NT_022184.14 Number 5323658) upstream of the exon 15, in the 14th intron of HADHB. This SNP was the intron SNP. Those having a minor allele A (healthy subjects 23+3=26, patients 15+0=15) were present at a significantly (p=0.0271) higher frequency in the patients compared with those not having the minor allele A (healthy subjects 73, patients 16), and its odds ratio was 2.63 times.

SNP having the genotypes of G/G, G/C and C/C at base number 1607 in HADHB was detected. This SNP was cSNP which did not affect the phenotype. Those having a minor allele C (healthy subjects 16+3=19, patients 13+1=14) were present at a significantly (p=0.0280) higher frequency in the patients compared with those not having the minor allele C (healthy subjects 86, patients 21), and its odds ratio was 3.47 times.

### 1-2. Genetic polymorphisms used for combination analysis of two or more genes

Only representative examples were listed below, and all polymorphisms detected are described in FIG. 4.

### [Genetic polymorphism observed in cording region]

Multiple cSNPs were detected in the 4th exon region (EXON 4) and the 5th exon region (EXON 5) of CPTII.

The SNP having the genotypes of T/T, T/G and G/G was detected at base number 1055. This SNP generates the phenotypes (F/F), (F/C) and (C/C) at amino acid level because the allele having T encodes phenylalanine (F) and the allele having G encodes cysteine (C) at position 352 in the amino acid sequence.

The SNP having the genotypes of G/G, G/A and A/A was detected at base number 1102. This SNP generates the phenotypes (V/V), (V/I) and (I/I) at amino acid level because the allele having G encodes valine (V) and the allele having A encodes isoleucine (I) at position 368 in the amino acid sequence.

The SNP having the genotypes of A/A, A/G and G/G was detected at base number 1939. This SNP generates the phenotypes (M/M), (M/V) and (V/V) at amino acid level because the allele having A encodes methionine (M) and the allele having G encodes valine (V) at position 647 in the amino acid sequence.

The cSNP was also observed in the ETF gene group. SNP having the genotypes of C/C and C/T was detected at base number 512 in ETFA. This SNP generates the phenotypes (T/T) and (T/I) at amino acid level because the allele having C encodes threonine (T) and the allele having T encodes isoleucine (I) at position 171 in the amino acid sequence.

The SNP having the genotypes of C/C, C/T and T/T was detected at base number 461 in ETFB. This SNP generates the phenotypes (T/T) (T/M) and (M/M) at amino acid level because the allele having C encodes threonine (T) and the allele having T encodes methionine (M) at position 154 in the amino acid sequence.

The SNP having the genotypes of C/C, C/T and T/T was detected at base number 447 in ETFB. This SNP was the SNP which had no effect on the phenotype.

The SNP having the genotypes of C/C, C/T and T/T was detected at base number 92 in ETFDH. This SNP generates the phenotypes (T/T) (T/I) and (I/I) at amino acid level because the allele having C encodes threonine (T) and the allele having T encodes isoleucine (I) at position 31 in the amino acid sequence.

The SNP having the genotypes of C/C, C/T and T/T was detected at base number -320 in ETFB, and further the NP having the genotypes of G/G and G/T was detected at base number -113 in ETFB.

The SNP having the genotypes of A/A, A/C and C/C was detected at base number 1989 in ETFDH. These SNPs were novel SNPs not reported conventionally in HGMD database and dbSNPs.

The SNP was detected in the ETFA intron region. The SNP having the genotypes of C/C, C/T and T/T was detected at position 548228 (corresponded to the 642nd base from the initiation position of the 10th exon in the coding sequence NT_024654.13, but no corresponding amino acid was present and thus this was thought to be included in the intron sequence). These SNPs were novel SNPs not reported conventionally in HGMD database and dbSNPs.

Each genetic polymorphism is often taken from a parent to a progeny together with the adjacent genetic polymorphism, and a relationship between those alleles is referred to as the linkage disequilibrium. The linkage disequilibrium tends to be weakened as the distance between the alleles gets longer. Therefore, in the proximal alleles, the strong linkage disequilibrium is often observed. When the allele shown herein and the adjacent allele are in the relationship of the strong linkage disequilibrium, the frequency and the appearance pattern of the genetic polymorphism become sometime the same, and it is possible to predict the genetic polymorphism of the present Example. Meanwhile, without limiting to the allele described herein, by analyzing the other adjacent allele, it becomes possible to utilize this for the risk diagnosis of the present invention. Even if there is the strong linkage disequilibrium, the risk diagnosis can be sometimes performed.

For example, in many cases, the genetic polymorphisms close to HADHA and HADHB are in linkage disequilibrium. Therefore, it is possible to perform the risk diagnosis by utilizing the genetic polymorphism adjacent to this gene.

### 3. Risk diagnosis by combination of SNPs in patients with influenza-associated encephalopathy (Table B)

### 3-1. Search of patient marker candidates

Subsequently, in order to utilize the genetic polymorphisms obtained above for the risk diagnosis, it was attempted to find the combination of the genetic polymorphisms observed only in the patients and the combination of the genetic polymorphisms frequently observed in the patients. The genetic polymorphism exhibiting the high odds ratio were obtained from the patient group by utilizing two or more genetic polymorphisms. Representative examples of the genetic polymorphisms were listed below.

The combination of the insertion having the genotypes of ACT/ACT or -/ACT (insertion of threonine) at base number 4 in the second exon of HADHB and the SNP having the genotypes of C/T or T/T in position -37 (NT_019273.17 number 16279498) in the 9th intron of HMGCS2 was observed in 5 of 27 specimens from the patients capable of being evaluated by the genetic analysis while only one in 99 specimens from the healthy subjects. The allele involving the position -37 in the 9th intron of HMGCS2 and the allele involving the position +68 (NT_019273.17 number 16279714)) in the 9th intron of HMGCS2 are in strong linkage disequilibrium. Therefore, when the genotype of G/G or A/G was combined with the genotype of ACT/ACT or -/ACT in the second exon of HADHB, the same result was obtained (observed in 5 of 27 specimens from the patients while one of 99 healthy subjects). The odds ratio is 22.4 times in both cases.

The combination of the genotype of C/T at base number 447 in the 8th exon of ETFB and the genotype of G/A at base number 1102 in the 4th exon of CPTII was observed in 2 of 31 specimens from the patients capable of being evaluated by the genetic analysis while no specimen of 100 healthy subjects.

The combination of the genotype of C/C at base number +642 in the 10th intron of ETFA and the genotype of G/T at base number -113 in the 3rd intron of ETFB was observed in 1 of 31 specimens from the patients capable of being evaluated by the genetic analysis while no specimen of 100 healthy subjects.

The combination of the genotype of T/G or G/G at position +33 downstream the 8th exon, in the 8th intron (NT_010718.15 number 6722966) of VLCAD and the genotype of A/A (phenotype of glutamine) at base number 997 in the 9th exon of LCAD was observed in 4 of 27 specimens from the patients capable of being evaluated by the genetic analysis while no specimen of 99 healthy subjects.

The combination of the genotype of C/C (phenotype of cysteine) at base number 1055 in the 4th exon of CPTII and the genotype of G/G or G/T at position +642 in the 10th intron of ETFA was observed in 9 of 31 specimens from the patients capable of being evaluated by the genetic analysis while 4 of 99 healthy subjects. In this case, the odds ratio was 9.72 times.

The combination of the genotype of ACT/ACT or -/ACT (insertion of threonine) at base number 4 in the 2nd exon of HADHB and the genotype of G/T at base number 1989 in the 13th exon of ETFDH was observed in 10 of 27 specimens from the patients capable of being evaluated by the genetic analysis while 12 of 99 healthy subjects.

A covering rate of the risk diagnosis in the patients can be increased by further putting the above combinations together. For example, when total 8 types of ETFB 447C/T, CPTII 1102G/A, ETFA +642C/C, ETFB -113G/T, VLCAD +33T/G or G/G, LCAD 997A/A, HADHB 4 -/ACT or ACT/ACT, and HMGCS2 -37C/T or T/T were used, 11 (the combination was duplicated in one) of 31 specimens from the patients satisfied these conditions. Meanwhile, only one of 99 specimens from the healthy subjects satisfied such conditions. This combination covered 35.5% of the patients, but was observed in only 1% of the healthy subjects (Table B(a)).

Alternatively, when total 6 types of HADHB 4 -/ACT or ACT/ACT, HMGCS2 -37C/T or T/T, VLCAD +33T/G or G/G, LCAD 997 A/A, ETFA642 C/C and CPTII 1055 G/G or G/T were combined, this combination was observed in 16 (duplicated combination in two) of 31 specimens from the patients and covered 51.6% of the patients while the combination was detected in 5 of 99 healthy subjects, which was 5.1% of the healthy subjects (Table B(b)).

Furthermore, when total 6 types of VLCAD +33T/G or G/G, LCAD 997A/A, HADHB1989 G/T, ETFDH 1989G/T, ETFA 642C/C and CPTII 1055G/G or G/T were used as another combination, this combination was observed in 22 (duplicated combination in three) of 31 specimens from the patients and covered 71% of the patients while this combination was detected in 14 (duplicated combination in one) of 99 healthy subjects, which was 13.1% of the healthy subjects (Table B(c)).

In the methods typified by the results shown above, the genetic polymorphisms can be used as markers for the risk diagnosis of the thermolabile phenotype diseases including influenza-associated encephalopathy.

### 3-2. Search of healthy subject marker candidates

By searching genetic polymorphism markers characteristic for the healthy subjects, it is possible to know whether a morbid risk is high or not, and it is thought that the morbid risk is low when not having such a genetic marker.

The combination of the genotype of A/C or C/C at base number 997 in the 9th exon of LCAD and the genotype of G/G at base number 2619 in the 18th exon of HADHA gene and the genotype of T/T at position +53 (NCBI) in the intron region of HMGCS2 was observed in 30 (30.3%) of 99 specimens from the healthy subjects capable of being evaluated by the genetic analysis while this combination was not observed at all in the patients (Table B(d)). From the above, this combination can be used as the risk diagnosis marker which has the small morbid risk.

**Table B**

| (a) | | | | | | |
|---|---|---|---|---|---|---|
| | | | | Genotype | patients | healthy |
| | ETFB | 447 | C>T | C/T | 2 | 0 |
| & | CPT2 | 1102 | G>A | G/A | | |
| | ETFA | +642 | C>T | C/C | 1 | 0 |
| & | ETFB | -113 | G>T | G/T | | |
| | VLCAD | +33 | T>G | T/G or G/G | 4 | 0 |
| & | LCAD | 997 | A>C | A/A | | |
| | HADHB | 4 | ->ACT | - /ACT or ACT/ACT | 5 | 1 |
| & | HMGCS2 | -37 | C>T | C/T or T/T | | |
| | | | TOTAL | | 11/31 | 1 /99 |
| | | | | | (35.5%) | (1.0%) |
| | | | | | | |
| b) | | | | | | |
| | | | | Genotype | patients | healthy |
| | HADHB | 4 | ->ACT | - /ACT or ACT/ACT | 5 | 1 |
| & | HMGCS2 | -37 | C>T | C/T or T/T | | |
| | VLCAD | +33 | T>G | T/G or G/G | 4 | 0 |
| & | LCAD | 997 | A>C | A/A | | |
| | ETFA | +642 | C>T | C/C | 9 | 4 |
| & | CPT2 | 1055 | T>G | G/G or GT | | |
| | | | TOTAL | | 16/31 | 5/99 |
| | | | | | (51.6%) | (5.1 %) |
| | | | | | | |
| c) | | | | | | |
| | | | | Genotype | patients | healthy |
| | VLCAD | +33 | T>G | T/G or G/G | 4 | 0 |
| & | LCAD | 997 | A>C | A/A | | |
| | HADHB | 4 | →ACT | - /ACT or ACT/ACT | 12 | 10 |
| & | ETFDH | 1989 | G>T | G/T | | |
| | ETFA | +642 | C>T | C/C | 9 | 4 |
| & | CPT2 | 1055 | T>G | G/G or G/T | | |
| | | | TOTAL | | 22/31 | 13/99 |
| | | | | | (71.0%) | (13.1 %) |
| | | | | | | |
| d) | | | | | | |
| | | | | genotype | patients | healthy |
| | LCAD | 997 | A>C | A/C or C/C | | |
| & | HADHA | 2619 | G>C | G/G | 0 | 30 |
| & | HMGCS2 | +53 | C>T | T/T | | |
| | | | TOTAL | | 0/27 | 30/99 |
| | | | | | 0% | 30.30% |

### Example 2

### Materials and Methods

### Patients

This study was approved by Ethical Review Board for human genome analysis in the University of Tokushima. The written informed consent was given to all participants. Surveillance for influenza-associated encephalopathy (IAE) was performed in influenza seasons in 2000 to 2003 in the southwest region in Japan. IAE was diagnosed according to clinical symptoms. All 34 patients had a viral antigen and exhibited sudden initiation of insult and coma which occurred 12 to 48 hours after running a high fever. In one death case (patient No. 21), diclofenac sodium was taken getting the fever, but fat denaturation in liver and typical pathological findings of Reye's syndrome were not observed. Thirteen IAE patients diagnosed as IAE including the patient No. 21 and 79 healthy volunteers agreed to be evaluated by genomic analysis.

### Clinical data analysis

Peripheral blood samples treated with EDTA, urine samples and samples obtained by cotton swab from throat were obtained from the patients. Organic acid profiles in urine and acylcarnitine in serum were analyzed by gas chromatography-mass spectrum (Shimadzu Model Qp5000, Shimadzu, Kyoto, Japan) and electrospray tandem mass spectrum (Model TSQ7000, Thermo-Quest, Tokyo, Japan). The influenza antigen was analyzed by enzyme linked immunosorbent assay (ELISA, Becton Dickinson) using the samples obtained by cotton swab from the throat.

### Assay for CPTII activity

A CPTII activity was measured by detecting palmitoyl-L-[methyl-³H]carnitine formed from L-[methyl-³H]carnitine and palmitoyl-CoA in COS-7 cells transfected with a CPTII cDNA mutant and liver biopsy samples in the presence of 1% Tween 20 in a reaction mixture. For analyzing the thermal stability of wild type CPTII and mutant CPTII, the activity in homogenates of the liver and the cells was measured at 37°C and 41°C.

### CPTII mutation analysis

Genomic DNA was purified from whole blood from IAE patients and 79 Japanese not having influenza, as described (Fukao T, Mitchell GA, Song X-Q, Nakamura H, Kassovska-Bratinova S, et al. Genomics 2000; 68: 144-51). Five exons of the CPTII gene were analyzed by PCR using primers based on introns (Table C). Sequences of PCR products were directly analyzed on ABI-PRISM 3100 Genetic Analyzer (PE-Applied Biosystems) using ABI DyeDeoxy Terminator Cycle Sequencing Kit. Each PCR product was sequenced for both strands, and the analysis was performed at least twice independently.

**Table C**

| Exon primers used for PCR amplification of CPTII gene; MA- or MB-primer was used for mutagenesis. | | | |
|---|---|---|---|
| Region | Forward primer | Reverse primer | Size of product (bp) |
| Exon 1 | cttgtgtttagactccagaactcc | gtcatgagtgactgcagtcaggttg | 292 |
| Exon 2 | ctgtcagccttacactgaccc | aactctcggggcttggtc | 305 |
| Exon 3 | Tttagggctatgctgttggg | aggaagggaggatgagacgt | 358 |
| Exon 4-1 | ctctggaggttgatgccatt | acccaagcactgaggacaag | 1,472 |
| Exon 4-2 | tagagttcagtgggtagctggct | atccaggcacatctgaagtac | 401 |
| Exon 5 | tttcctgaggtccttttccatcctg | atgaggaagtgatggtagcttttca | 425 |
| MA-V352I | ggcacaaaccgctggtgtgataaat | atttatcacaccagcggtttgtgc | |
| MB-V362I | ctactgccgtccactttagcactcttg | caagagtgctcaaagtggacggcagtag | |

### Mutagenesis and expression of mutant CPTII and WT CPTII in COS-7 cells

A full length CDNA clone (pCMV6-WT) including total coding regions of human CPTII was gifted by V. Esser, PhD, the University of Texas. The plasmid pCMV6-WT was used as a parent vector for generating three full length mutant CPTII cDNA clones, pCMV6-MA (F352C), pCMV6-MB (V362I) and pCMV6-MA+B (F352C/V362I) using QuickChange (registered trade name) (site-directed mutagenesis Kit) (Stratagene). The primers used for mutagenesis are listed in Table C. Mutation of CPTII cDNA and perfectibility thereof were identified by sequence analysis. As an internal standard for monitoring a transfection efficiency by measuring the enzyme activity, pSVb-galactosidase control vector (Promega) was transfected simultaneously with various pCMV6-CPTII plasmids. Mock transfection was performed as the control. COS-7 cells were washed twice with saline 72 hours after the transfection, and the activity of WT and mutant CPTII was analyzed.

### Results

### Patients and acylcarnitine ratio

Thirty-four patients (22 boys and 12 girls) aged 0 to 16 with no potential disease had IAE. Twenty-two patients (64.7%) were younger than 4 years old, and the average age was 2 years old. Influenza A, B and A+B viral antigens were detected by nasal throat cotton swab in 91.2%, 5.9% and 2.9%, respectively. Some patients (2.9%) were already vaccinated. The patients except for one lethal case (patient No. 21) did not take aspirin or diclofenac during the course of influenza.

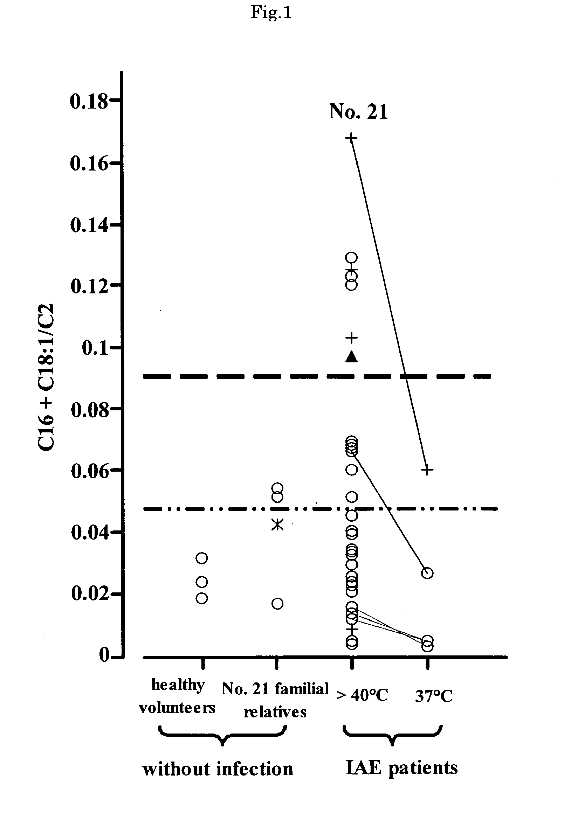

In laboratory experiments using the sera from the patients, it was demonstrated that 41.2% of the IAE patients exhibited characteristic elevation of serum acylcarnitine ratio (C_{16:0}+C_{18:1})/C₂ which exceeds 0.048 which was an upper limit of a cutoff value (FIG. 1). In particular, a half or more of the patients with severe IAE (7 patients) having this ratio of more than 0.09 became lethal (3 girls) and had aftereffects (one boy). These data indicates that the patient with high risk has a remarkable disturbance of long-chain fatty acid metabolism in mitochondria, but no symptom showing the abnormality was not observed in the diagnosis of these patients before the onset. In Japanese, there are two types of the most typical congenital anomaly in fatty acid β oxidation metabolism in mitochondria. One is CPTII deficiency, which exhibits the accumulation of long-chain acylcarnitine in serum. Another type is glutaric acid acidic urine type 2 (GA2). They exhibit the frequencies of 26.6% and 21.9%, respectively. In Caucasian, these frequencies are 11% and 5.5%, respectively which are relatively low. The most typical deficiency in Caucasian is the deficiency of medium-chain acyl-CoA dehydrogenase (MCAD), which is 27.5% in frequency, the highest frequency. However, in all IEA patients, when the body temperature becomes normal, the (C_{16:0}+C_{18:1}) /C₂ ratio is reduced to a birder line ratio of 0.048 to 0.06 or normalized. These results suggest that the ratio was temporally elevated upon convulsion with fever at 40°C or higher. All family members except for a mother of the patient number 21 having the combination of thermolabile CPTII polymorphisms exhibited borderline ratios of 0.042 to 0.054 in an uninfected state as described below. An elder sister having the same combination of CPTII polymorphisms as in the patient number 21 exhibited the ratio of 0.42 at normal body temperature. Even when two patients were in the high risk group having the ratio of more than 0.09, they recovered 3 to 4 weeks after the infection without having the aftereffect.

One lethal IAE patient having the ratio of 0.004 was present (FIG. 1), and this patient exhibited the abnormal increase of glutaric acid in urine. That is, this is the death case diagnosed as GA2 based on electron transfer disturbance in the mitochondria. All of the IAE patients having the ratio of 0.09 or lower recovered without having the severe aftereffect. Octanoylcarnitine which is the diagnostic marker of MCAD deficiency was not elevated in all of the IAE patients examined in Japan.

### Thermolabile CPTII polymorphism

A CPTII specific activity in the homogenate of liver biopsy in the patient number 21 having the highest acylcarnitine ratio of 0.168 was 0.4 ± 0.06 nmol/min/mg protein, which was about 36% of the normal control (1.1 ± 0.3 nmol/min/mg protein n=6) at 37°C.

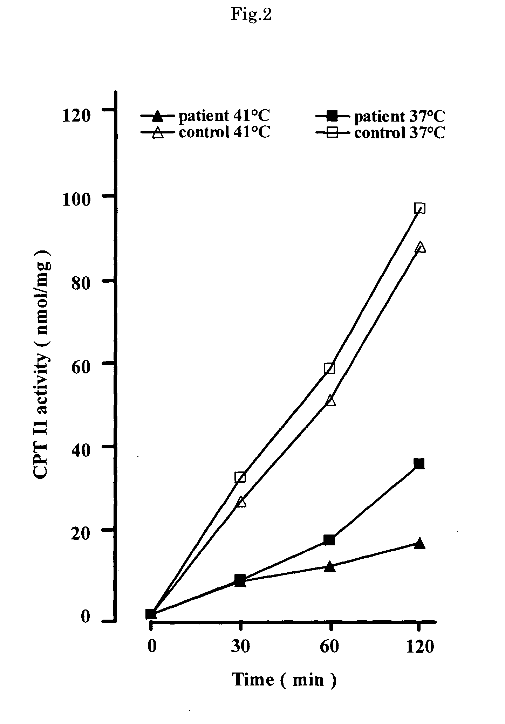

CPTII in this patient was extremely thermolabile, and its specific activity was reduced to about 50% after incubation at 41°C for 120 minutes, but it is noteworthy that the specific activity of the control CPTII was slightly reduced to 91.4% under the same assay condition (FIG. 2). In order to analyze causal factors of thermolabile property of CPTII in the patient, the present inventor analyzed the genotypes in the patient number 21 and the family thereof. The sequence analysis of the CPTII gene has revealed that the patient has heterozygous polymorphism, i.e., F352C substitution and V368I substitution and other reported CPTII mutations were not detected. The F352C polymorphism mutant has been reported only in Japanese, and has not been reported in Caucasian. The V368I mutant has been found in both Japanese and Caucasian, and the CPTII deficiency is discussed only for the possibility of its relatively mild harmful effect. The patient number 21 and its elder sister had the same heterozygote for F352C substitution and V368I substitution. Its father had homozygote for F352C substitution and V368I substitution. Its mother had heterozygote only for V368I substitution. Its elder brother had the heterozygote for F352C substitution and the homozygote for V368I substitution. No other substitution in CPTII was identified in the family and its relatives. The substitution M647V reported was found in some IAE patients, but no significant difference between its frequencies in the IAE patients and the healthy volunteers was observed. In the comparison of frequencies of CPTII haplotypes between the IAE patients and the healthy volunteers (type 1 to 9 in Table 3), the type 9 is matched to the combination of F352C, V368I and M647M which is identical to the genotypes in the patient number 21, and its frequency in the patients is significantly higher than that in the healthy volunteers (p<0.025). Due to the limited number of the patients analyzed, no other haplotype whose frequency was significantly higher in the IAE patients than in the healthy volunteers was observed at present (Table D).

**Table D**

| CPTII haplotypes and their frequencies in IAE patients and healthy volunteers | | | | | |
|---|---|---|---|---|---|
| Haplotype | Patients | | Healthy volunteers | | P |
| | Number | Frequency (%) | Number | Frequency (%) | |
| Type 1 (F/F-V/V-M/M) | 0 | 0 | 11 | 13.9 | |
| Type 2 (F/F-I/I-V/V) | 0 | 0 | 1 | 1.2 | |
| Type 3 (F/F-V/I-M/V) | 1 | 7.7 | 3 | 3.8 | |
| Type 4 (F/F -I/I-M/V) | 1 | 7.7 | 1 | 1.2 | |
| Type 5 (F/F-I/I-M/M) | 3 | 23.1 | 25 | 31.6 | |
| Type 6 (F/F-V/I-M/M) | 2 | 15.4 | 27 | 34.2 | |
| Type 7 (C/C-I/I-M/M) | 1 | 7.7 | 5 | 6.3 | |
| Type 8 (F/C-I/I-M/M) | 1 | 7.7 | 0 | 0 | |
| Type 9 (F/C-V/I-M/M) | 4 | 30.8 | 6 | 7.6 | <0.025 |
| Total number | 13 | | 79 | | |

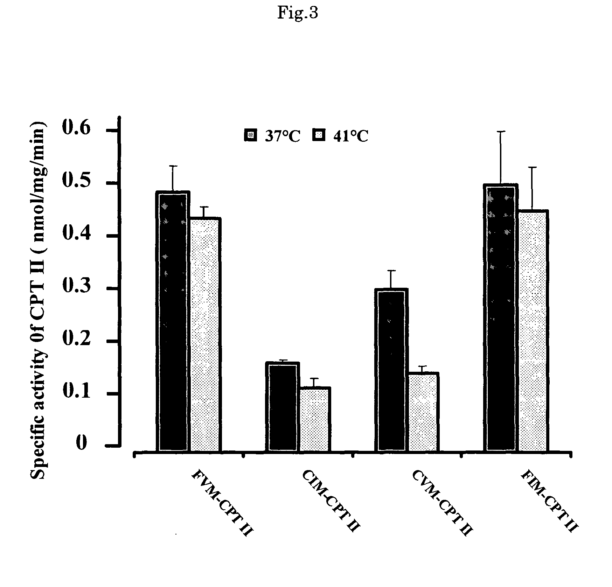

For the expression *in vitro* of WT and mutant CPTII cDNA, 4 types of cDNAs were excessively expressed in COS-7 cells (FIG. 3): pCMV6-WT includes WT CPTII (FVM-CPTII); pCMV6-MA includes F352C polymorphism (CVM-CPTII); pCMV6-MB includes V362I polymorphism (FIM-CPTII); and pCMV6-MA+B includes F352C+V362I polymorphisms (CIM-CPTII). The CPTII activity in the vector alone was 0.27 ± 0.03 nmol/min/mg protein (n=6), which is identical to an endogenous enzyme activity. Thus all data shown below are those obtained by subtracting the value of the CPTII activity in the vector alone. The CPTII activity in COS-7 cells in which pCMV6-WT had been excessively expressed was 0.46 ± 0.07 nmol/min/mg protein (n=5). The CPTII activities obtained from CVM-CPTII, FIM-CPTII and CIM-CPTII were 62.8 ± 7.2%, 102.5 ± 19.6% and 34.7 ± 1.3% of the WT FVM-CPTII activity at 37°C (FIG. 3). The amino acid substitution potentially affects the conformational change and the thermal stability of the protein. Thus, the present inventor analyzed the CPTII activity under the heat stress condition at 41°C as a representative example of the temperature at 39°C or higher. The activity of WT FVM-CPTII was slightly reduced to 91% after the incubation at 41°C for 120 minutes. The activities of FIM-CPTII, CVM-CPTII and CIM-CPTII were reduced to 91%, 48% and 72%, respectively at 41°C compared with the activities at 37°C. In these mutants, the enzyme activities of both CIM-CPTII and CVM-CPTII were remarkably reduced to about 25 to 30% of the WT FVM-CPTII activity at 37°C.

In this experimental example, the decrease of the enzyme activity at 41°C was examined, but the thermolabile phenotype disease can be diagnosed by likewise examining the significant decrease of the enzyme activity at optional temperature (e.g., 39°C, 40°C, 42°C) of 39°C or higher.

These results are derived from the substitution of CPTII homozygote, and these data support the decrease of the CPTII activity observed in the patient number 21 at 41°C in FIG. 2. Discussion

The encephalitis/encephalopathy is one of symptoms which appear in various juvenile diseases. Among them, IAE has been reported as the complication of influenza and to be observed at high frequency paricularly in Japanese children. In the present invention, the present inventor has revealed that 41.2% of the IAE patients exhibited the elevation of the serum acylcarnitine ratio (C_{16:0}+C_{18:1})/C₂ which exceeded the cutoff value upper limit of 0.048 during the febrile convulsion and that 57% of the patient in high risk having the ratio of more than 0.09 clinically resulted in death or having the severe aftereffect. This ratio is the marker for the long-chain fatty acid metabolism disturbance. The most common disease exhibiting this type of the phenotype in Japan is the CPTII deficiency which is the congenital abnormality and is the extremely rare disease having its frequency of several per 0.1 million of population. It is important to remember that the abnormal values of acylcarnitine are detected in 41.2% of the patients diagnosed as IAE. In the group researched, GA2 in addition to the CPTII deficiency also induces the lethal cause disease, the electron transfer in the mitochondria is impaired in this disease, and the patient had the acylcarnitine ratio of less than 0.048. From these results, all of the severe IAE patients resulted in death (4 patients) and having the severe aftereffect (one patient) had the disturbance of mitochondrial energy metabolism, e.g., abnormal CPTII and GA2. The acylcarnitine ratio elevated with high fever was significantly decreased when the body temperature went back to normal in all of the followed patients, suggesting that the thermolabile phenotype is reversible, which is noteworthy. These findings indicate that the continuous high fever is often accompanied by emesis and fasting and in particular systemic energy crisis is caused in the patient having energy metabolism enzyme deficiency or thermolabile polymorphism.

The children having the congenital abnormality of mitochondrial fatty acid β oxidation such as CPTII deficiency and GA2 in Japanese and MCAD deficiency in Caucasian have severe acute encephalopathy and multiple organ failure (Reye's syndrome) without having the infection. Of course, when these children exhibiting the congenital abnormality are infected with influenza, they highly likely result in becoming severe leading to death. The data of the present application were from the cases where the IEA patients even having the high acylcarnitine ratio did not have these enzyme deficiency and had multiple thermolabile polymorphisms or CPTII thermolabile polymorphisms. These polymorphisms synergistically decrease the enzyme activity. Almost all of the IAE patients did not exhibit a clear episode, and thus, has escaped from the detection because no symptom is exhibited in neonatal phase. Reye's syndrome caused by dosing aspirin together with the infection is potentially developed in the patient having the genetic background similar to the enzyme involved in mitochondrial fatty acid β oxidation, but pathological findings in the liver exhibits stronger fat denaturation than that observed in IAE.

The present inventor has demonstrated that the functional deficiency of CPTII is synergistically induced in the feverish condition by combining CPTII thermolabile F352C polymorphism with the specific mildly harmful V3681 polymorphism, and proposed that these polymorphisms were made the mutants which easily induce IAE. The polymorphisms V368I and M647V have been reported in the genetic polymorphisms of the patients with CPTII deficiency and asymptomatic juveniles in both Japanese and Caucasian. However, CPTII is not a rate limiting enzyme in the metabolic pathway of β oxidation, and when the CPTII activity is more than 30% of the control, the fatty acid β oxidation is in normal range and no abnormal symptom appears. However, the CPTII activity from the patient number 21 and the activities of transfected CIM-CPTII and CVM-CPTII at 39°C or higher, e.g., under the condition of heat stress at 41°C were less than 30% of the WT-CPTII activity at 37°C, presuming that these exhibited the fatty acid β oxidation disturbance. Concerning three CPTII polymorphisms such as F352C, V368I and M647V, 27 haplotypes are predicted. Among them, 9 haplotypes as shown in Table 3 were observed in the IAE patients and the healthy volunteers. In these, the frequency of the type 9 (F/C-V/I-M/M) in the IAE patients was significantly higher than in the healthy volunteers. CIM-CPTII (type 7, C/C-I/I-M/M) exhibited the lowest enzyme activity in FIG. 3, but the difference between the frequencies of the type 7 in the IAE patients and the healthy volunteers was not significant for the moment, and this seemed to be attributed to limited number of the analyzed IAE patients. Furthermore, these findings do not deny the potential involvement of the deficiency or the mild mutation of the other enzyme involved in the energy metabolism in the thermolabile polymorphism and/or the causal factors of the thermolabile phenotype disease. Molecular mechanisms for encephalopathy and acute cerebral edema observed in the IAE patient having the disturbance of mitochondrial β oxidation have not been elucidated. However, there is a possibility that the accumulation of miniplasmin in cerebral blood capillary in mice having congenital or acquired abnormality of the mitochondrial β oxidation and the breakdown of miniplasmin by protease in blood brain barrier after the infection with influenza virus are proved as the related causal factors.

Considering the energy metabolism abnormality in the IAE patient having the thermolabile polymorphism, the administration of L-carnitine and glucose for activating the long-chain fatty acid oxidation and further therapeutic hypothermia for preventing the enzyme deactivation induced by high fever may be proved to be therapeutically effective in the case of the thermolabile phenotype disease.

### INDUSTRIAL APPLICABILITY

1. For the disease leading to death with cerebral edema and multiple organ failure with high fever, whose cause has been described to be unknown, it becomes possible to identify the disease susceptible gene to be the preposition of the disease and diagnose it definitely.
2. It becomes possible to develop the therapeutic method based on the identification of the disease susceptible gene to be the preposition of the disease.

Furthermore, the present invention is thought to be the technology which enables to determine whether having the high risk to have the thermolabile phenotype disease in the future or not by examining if having the SNP or not.

## Claims

1. A method of diagnosing a risk of a thermolabile phenotype disease including or caused by influenza encephalitis/encephalopathy, Reye's syndrome, RS virus infectious disease, adenovirus infectious disease, rhinovirus infectious diseases, bastard measles, Japanese encephalitis, malaria infectious disease, Kawasaki disease and sudden infant death syndrome, **characterized by** examining whether or not an enzymatic activity of at least one enzyme involved in any of various transporters, carnitine cycle, long-chain β oxidation cycle, medium-chain/short-chain β oxidation cycle, electron transfer, synthesis of a ketone and production of ATP involved in energy metabolism in mitochondria is significantly lower compared with healthy subjects at 39°C or higher when referring the enzymatic activity at 37°C as to 100%.

2. The method according to claim 1 wherein said thermolabile phenotype disease is influenza encephalitis/encephalopathy.

3. The method according to claim 1 **characterized in that** a degree of said decreased enzymatic activity at 39°C compared with said enzymatic activity at 37°C is forecasted by examining polymorphism selected from the group consisting of SNP, insertion and deletion in a gene encoding said enzyme.

4. The method according to claim 1 wherein the enzyme involved in any of various transporters, carnitine cycle, long-chain β oxidation cycle, medium-chain/short-chain β oxidation cycle, electron transfer, synthesis of a ketone and Production of ATP is at least one enzyme shown in the following Table. [Table 1]

5. The method according to claim 4 wherein said enzyme is at least one selected from the group consisting of CPTII, ETFA, ETFB, ETFDH, HADHB, HMGCS, VLCAD, LCAD and HADHA.

6. The method according to claim 1 **characterized in that** concerning CPTII, ETFA, ETFB, HADHA, HADHB, HMGCS, VLCAD, LCAD and ETFDH, SNP of genes at one or two positions in the following 22 positions or genes being in linkage disequilibrium therewith is examined and a combination of two or more genetic polymorphisms or haplotypes is utilized.
**Table 2**
| Enzyme name | Position of SNP | |
|---|---|---|
| CPT2 | (EXON4) | 1055 |
| CPT2 | (EXON4) | 1102 |
| ETFA | (INTRON10) | +642 |
| ETFB | (EXON1) | -320 |
| ETFB | (EXON3) | -113 |
| ETFB | (EXON8) | 447 |
| ETFB | (EXON8) | 461 |
| HADHA | (EXON6) | 474 |
| HADHA | (INTRON6) | +26 |
| HADHA | (INTRON6) | +32 |
| HADHA | (EXON18) | 2519 |
| HADHA | (EXON18) | 2619 |
| HADHB | (EXON2) | 4 |
| HADHB | (INTRON12) | -14 |
| HADHB | (INTRON14) | +4 |
| HADHB | (INTRON14) | -26 |
| HADHB | (EXON17) | 1607 |
| HMGCS | (INTRON8) | -37 |
| HMGCS | (INTRON8) | +53 |
| VLCAD | (INTRON8) | +33 |
| LCAD | (EXON9) | 997 |
| ETFDH | (EXON13) | 1989 |

7. The method according to claim 1 wherein the risk of the thermolabile phenotype disease is diagnosed based on whether having the combination of any SNP in the following (1) to (8) or not.
**Table 3**
| Combination | Enzyme | Polymorphism position (base number) | Genotype | Risk |
|---|---|---|---|---|
| (1) | ETFB | 447 | C/T | Large |
| | CPT2 | 1102 | G/A | |
| (2) | ETFA | +642 | C/C | Large |
| | ETFB | -113 | G/T | |
| (3) | ETFA | +642 | C/C | Large |
| | CPT2 | 1055 | G/G or G/T | |
| (4) | HADHB | 4 | -/ACT or ACT/ACT | Large |
| | HMGCS | -37 | C/T or T/T | |
| (5) | VLCAD | +33 | T/G or G/G | Large |
| | LCAD | 997 | A/A | |
| (6) | CPT2 | 1055 | T/G or G/G | Large |
| | CPT2 | 1102 | G/A or A/A | |
| (7) | HADHB | 4 | -/ACT or ACT/ACT | Large |
| | ETFDH | 1989 | G/T | |
| (8) | LCAD | 997 | A/C or C/C | Small |
| | HADHA | 2619 | G/G | |
| | HMGCS | +53 | T/T | |

8. The method according to any of claims 1 to 7 wherein single nucleotide polymorphism (SNP) is detected by at least one method selected from the group consisting of a nucleotide direct base sequencing method, an allele specific oligonucleotide (ASO)-dot blotting analysis, a single base primer extension method, a PCR-single strand conformation polymorphism (SSCP) analysis, a PCR-restriction enzyme fragment length polymorphism (RFLP) analysis, an invader method, a quantitative real-time detection method and a single nucleotide polymorphism detection method (mass array) using a mass spectrometer.

9. The method according to claim 3 **characterized in that** the polymorphism selected from the group consisting of SNP, insertion and deletion in the gene encoding said enzyme is measured using a solid phase support to which at least one corresponding probe has been immobilized.

10. A diagnostic kit for diagnosing a risk of a thermolabile phenotype disease comprising primers, probes, a dNTP mix, reverse transcriptase, DNA polymerase and buffer capable of detecting one or a combination of two or more specific polymorphisms of an enzyme involved in β oxidation fatty acid metabolic system in mitochondria.
